# EUROPEAN PATENT APPLICATION

(11) **EP 4 667 033 A1**
(43) Date of publication of application: **24.12.2025**
(21) Application number: 24305978.9
(22) Date of filing: 20.06.2024
(51) Int. Cl.: A61M 5/32

(54) **HOUSING DEVICE AND MANUAL INJECTION DEVICE INCLUDING THE HOUSING DEVICE**

(71) Applicant: Becton Dickinson France, 38800 Le Pont-de-Claix (FR)
(72) Inventor: BESSON, Nicolas, 38650 TREFFORT (FR); DELOBELLE, Vincent, 38420 DOMENE (FR); ALVAIN, Olivier, 38180 SEYSSINS (FR); RADIC, Marijan, 174 41 SUNDBYBERG (SE)
(74) Representative: Germain Maureau

(57) **Abstract**

This housing device (10) is configured to receive a medical container (100) having an injection needle (103) and a needle shield (104) for sealing the injection needle (103). The housing device (10) includes: a body (6) extending along a longitudinal axis A, the body (6) delimiting an inner cavity for accommodating the medical container (100); a cap (7) removably attached to the body (6), the cap (7) being rotationally movable around the longitudinal axis A with respect to the body (6) between an initial position and a release position; a reversible remover (8) coupled to the cap (7), the reversible remover (8) including a removing member (88) configured for removing the needle shield (104) from the medical container (100) and releasing the needle shield (104) back onto the medical container (100), the removing member (88) being radially movable between a removing position and a release position, in which the removing member (88) releases the needle shield (104); and a cam element (61) arranged on the body (6) for cooperating with a cam member (85) of the remover (8) such that rotation of the cap (7) with respect to the body (6) from the initial position to the release position causes movement of the removing member (88) from the release position to the locking position, and rotation of the cap (7) with respect to the body (6) from the release position to the initial position causes movement of the removing member (88) from the locking position to the release position.

## Description

The present invention relates to a housing device and a manual injection device including this housing device.

In this application, the distal end of a component or of a device is to be understood as meaning the end furthest from the user's hand and the proximal end is to be understood as meaning the end closest to the user's hand during pricking and injection. Likewise, in this application, the "distal direction" is to be understood as meaning the direction away from the user's hand, and the "proximal direction" is to be understood as meaning the direction toward the user's hand.

Injection devices usually include a medical container provided with an injection needle initially covered by a needle shield and arranged within a housing for allowing a user to handle the injection device and perform an injection operation.

Before injection, the needle shield is removed from the medical container to unveil the injection needle, thus permitting injection. After injection, the needle shield is typically discarded without being re-attached to the medical container, and the injection needle is typically covered by a needle cover which extends from within the housing to protect the user against needle stick injuries. Then, the housing, the needle cover, and the used medical container are altogether disposed in a sharps container. Thereore, the housing, the needle cover and all associated features can never be reused.

There is therefore a need for an injection device that alleviates some or all of the aforementioned drawbacks of the prior art, and that is more sustainable while efficiently protecting the user against needle stick injuries. Specifically, there is a need for an injection device that can be reused, except for the used medical container that was in contact with the drug.

In this context, an aspect of the invention is a housing device configured to receive a medical container having an injection needle and a needle shield for sealing the injection needle, the housing device including:
a body extending along a longitudinal axis A, the body delimiting an inner cavity for accommodating the medical container,
a cap removably attached to the body, the cap being rotationally movable around the longitudinal axis A with respect to the body between an initial position, in which the cap is axially locked to the body, and a release position, in which the cap can distally move with respect to the body in order to be removed,
a reversible remover coupled to the cap, the reversible remover including a removing member configured for removing the needle shield from the medical container and releasing the needle shield back onto the medical container, the removing member being radially movable between a removing position, in which the removing member is configured for removing the needle shield, and a release position, in which the removing member releases the needle shield,
a cam element arranged on the body for cooperating with a cam member of the remover such that rotation of the cap with respect to the body from the initial position to the release position causes movement of the removing member from the release position to the locking position, and rotation of the cap with respect to the body from the release position to the initial position causes movement of the removing member from the locking position to the release position.

Therefore, the device of the invention permits to reposition the needle shield on the injection needle of a used medical container, i.e. after injection. This allows for a safe disposal of the used medical container in an appropriate waste container. Besides, the housing device can then be re-used in association with a new medical container, thus improving sustainability.

The remover may be configured for gripping or clamping the needle shield.

The remover is a reversible remover because the remover, together with the cap, can remove and subsequently put the needle shield back on the distal tip of the medical container.

In the removing position, the remover is configured for distally abutting against the needle shield such that distal movement of the reversible remover together with the cap causes distal movement of the needle shield, and thus removal of the needle shield from the medical container.

The reversible remover may be rotationally fixed to the cap, such that rotation of the cap causes rotation of the reversible remover with respect to the body. That is, the reversible remover rotates together with the cap. The reversible remover may be arranged within the cap.

The release position of the reversible remover may be centrifugally located with respect to its locking position.

The housing device may include a reversible remover coupled to the cap and configured for removing the needle shield of the medical container and putting the needle shield back on the medical container, the remover being radially movable between a removing position, in which the remover is configured for removing the needle shield, and a release position, in which the remover releases the needle shield, and a cam element arranged on the body for cooperating with a cam member of the remover such that rotation of the cap with respect to the body from the initial position to the release position causes movement of the remover from the release position to the locking position, and rotation of the cap with respect to the body from the release position to the initial position causes movement of the remover from the locking position to the release position.

The cap may rotate in one way from the initial position to the release position to cause movement of the remover from the release position to the locking position, and may rotate in the opposite way to cause movement of the reversible remover from the locking position to the release position. The initial position and the release position of the cap may be separated by a 70° to 110° angle, for instance a 90° angle.

The device of the invention may further include some or all of the features listed below.

In an embodiment, the housing device includes a holder including a proximal abutment configured for axially abutting against the medical container, the holder being axially movable along the longitudinal axis A with respect to the body between an initial position and an injection position, distally located with respect to the initial position.

Preferably, the housing device includes a safety spring for moving the holder towards the initial position.

In an embodiment, the holder includes an injection depth controlling member configured for axially abutting against the body to stop distal movement of the holder at a predetermined axial position.

In this predtermined axial position, the injection needle reaches a targeted injection depth inside the injection site.

In an embodiment, the holder includes an inner ring for receiving the medical container, and the inner ring has a distal end provided with a proximally tapered surface for avoiding that the needle shield catches on the inner ring when the used medical container is being removed from the housing device.

In an embodiment, the housing device includes a blocking element movable between a blocking position in which the blocking element axially abuts against the holder to prevent distal movement of the holder during the cap removal, and a release position, in which the blocking element allows for distal movement of the holder with respect to the body.

In an embodiment, the housing device includes a locker configured for axially locking the medical container within the body.

In an embodiment, the locker is movable with respect to the body between an initial release position, in which the locker allows for insertion of the medical container within the body, and a locking position, in which the locker prevents removal of the medical container from the body.

In an embodiment, the locker is rotationally movable between the initial release position and the locking position, and the housing device includes a driving member coupled to the cap such that rotation of the cap from the initial position to the release position causes rotation of the locker from the release position to the locking position, and rotation of the cap from the release position back to the locking position causes rotation of the locker from the locking position back to the initial release position.

The driving member is coupled to the pin of the reversible remover arranged in the cap. Thus, the driving member is indirectly coupled to the cap.

In an embodiment, the driving member is arranged on the reversible remover.

In an embodiment, the locker includes a dampener for reducing impact of the medical container against the locker when the cap is removed from the body.

In an embodiment, the housing device includes one or more retaining members configured to retain the locker in the initial release position and in the locking position.

The retaining members avoid inadvertent rotation of the locker, but allow for rotation of the locker when the cap is rotated by the user, i.e. when a predetermined torque is applied to the locker via the cap.

The blocking element may be radially movable. The release position may be radially inwardly located with respect to the locking position. The blocking element may be arranged on the locker or on the body.

In an embodiment, the housing device includes a shell configured for allowing a user to handle the housing device.

In an embodiment, the housing device includes a proximal stop configured for axially abutting against the needle shield in order to stop insertion of the medical container at a predetermined axial position.

In this predetermined axial position, the reversible remover is able to reliably engage the needle shield. In this predetermined axial position, a radial flange of the medical container may be axially distant from the holder by a first axial clearance, and may be distant from the locker by a second axial distance.

In an embodiment, the housing device includes snap-fit elements for maintaining the reversible remover in the removing position.

Possibly, the housing device include biasing means for moving the reversible remover towards the locking position.

According to an embodiment, the cam element includes a circumferential slot. The circumferential slot may be provided through a functional flange of the body and may be configured for causing movement of the reversible remover in one way or the other. Possibly, the circumferential slot includes a locking end shaped to forbid axial withdrawal of a pin of the reversible remover, and a releasing end larger than the locking end such that the releasing end is shaped to allow axial insertion or extraction of said pin inside or outside the circumferential slot. The circumferential slot includes a camming surface which may converge towards the central longitudinal axis A from the locking end to the releasing end such that the pin moving inside the circumferential slot is pushed towards the central longitudinal axis A when the cap is moved from the locking to the release position. Facing this camming surface, another camming surface may diverge from the central longitudinal axis A from the releasing end to the locking end such that the pin moving inside the circumferential slot is pushed away from the central longitudinal axis A when the cap is moved from the release to the locking position. The circumferential slot may be configured such that the pin is only released when reaching the releasing end.

Another aspect of the invention is an injection device including the aforementioned housing device and a medical container arranged within the housing device.

The invention and the advantages arising therefrom will clearly emerge from the detailed description that is given below with reference to the appended drawings as follows :
- Figure 1A is a perspective view of an assembled injection device according to an embodiment of the invention,
- Figure 1B is a perspective view of an disassembled injection device according to an embodiment of the invention,
- Figure 1C is an exploded view of a housing device according to an embodiment of the invention,
- Figures 2A, 2B are 90° cross-section views of a shell of a housing device according to an embodiment of the invention,
- Figure 2C is a perspective view of a shell of a housing device according to an embodiment of the invention,
- Figure 3A is a perspective view of a body of a housing device according to an embodiment of the invention,
- Figure 3B is a perspective cross-section view of a body of a housing device according to an embodiment of the invention,
- Figure 3C is a top view of a body of a housing device according to an embodiment of the invention,
- Figure 4A is a perspective view of a locker of a housing device according to an embodiment of the invention,
- Figures 4B, 4C are 90° cross-section views of a locker of a housing device according to an embodiment of the invention,
- Figures 5A, 5B are, respectively, a perspective view and a cross-section view of a holder of a housing device according to an embodiment of the invention,
- Figures 6A, 6B, 6C are perspective views of a cap of a housing device according to an embodiment of the invention,
- Figure 7 is a perspective view of a reversible remover of a housing device according to an embodiment of the invention,
- Figures 8, 9 are perspective views of an injection device according to an embodiment of the invention,
- Figure 10 is a perspective view of an injection device according to an embodiment of the invention, the shell and the locker having been withdrawn for the sake of clarity,
- Figure 11 is a cross-section view of a portion of an injection device according to an embodiment of the invention,
- Figures 12A, 12B are 90° cross-section views of an injection device according to an embodiment of the invention,
- Figure 13 is a perspective view of an injection device according to an embodiment of the invention,
- Figure 14 is a perspetive view of an injection device according to an embodiment of the invention, the shell and the locker being withdrawn for the sake of clarity,
- Figure 15 is a cross section view of an injection device according to an embodiment of the invention,
- Figure 16 is a bottom view of a part of an injection device according to an embodiment of the invention,
- Figures 17, 18 are perspective views of portions of an injection device according to an embodiment of the invention,
- Figure 19 is a cross-section view of an injection device according to an embodiment of the invention,
- Figure 20 is perspective view of a cap of an injection device according to an embodiment of the invention,
- Figures 21, 22, 23, 24 are cross-section views of an injection device according to an embodiment of the invention,
- Figure 25 is a perspective view of a portion of an injection device according to an embodiment of the invention,
- Figures 26, 27, 28 are cross-section view of an injection device according to an embodiment of the invention,
- Figures 29, 30 are perspective views of a portion of an injection device according to an embodiment of the invention,
- Figure 31 is a cross-section view of a portion of an injection device according to an embodiment of the invention,
- Figure 32 is a perspective view of a portion of an injection device according to an embodiment of the invention,
- Figure 33 illustrates removal of a used medical container from a housing device according to an embodiment of the invention and disposal of said used medical container in a sharps container,
- Figures 34A-34D illustrate an injection device according to another embodiment of the invention,
- Figure 35 illustrates an injection device according to another embodiment of the invention,
- Figures 36A-36D illustrate an injection device according to another embodiment of the invention,
- Figures 37A-37D illustrate an injection device according to another embodiment of the invention,
- Figures 38A-38C illustrate an injection device according to another embodiment of the invention,
- Figures 39A-39B illustrate an injection device according to another embodiment of the invention,
- Figures 40A-40C illustrate an injection device according to another embodiment of the invention,
- Figures 41A-41D illustrate an injection device according to another embodiment of the invention,
- Figures 42A-42D illustrate an injection device according to another embodiment of the invention,
- Figures 43A-43G illustrate an injection device according to another embodiment of the invention,
- Figures 44A-44F illustrate an injection device according to another embodiment of the invention,
- Figures 45A-45E illustrate an injection device according to another embodiment of the invention,
- Figures 46A-46C illustrate an injection device according to another embodiment of the invention.

The different features of the embodiments can be used in combination with and used with other embodiments as long as the combined parts are not inconsistent with or interfere with the operation of the device and assembly. This invention is not limited in its application to the details of construction and the arrangement of components set forth in the following description or illustrated in the drawings. The embodiments herein are capable of being modified, practiced or carried out in various ways. The phraseology and terminology used herein is for the purpose of description and should not be regarded as limiting. The use of "including," "comprising," or "having" and variations thereof herein is to encompass the items listed thereafter and equivalents thereof as well as additional items. Unless limited otherwise, the terms "connected," "coupled," and "mounted," and variations thereof herein are used broadly and encompass direct and indirect connections, couplings, and mountings. In addition, the terms "connected" and "coupled" and variations thereof are not limited to physical or mechanical connections or couplings. In contrast, when an element is referred to as being "directly connected" or "directly coupled" to another element, there are no intervening elements present. Unless otherwise defined, all terms (including technical and scientific terms) used herein have the same meaning as commonly understood by one of ordinary skill in the art to which this disclosure belongs. It will be further understood that terms used herein should be interpreted as having a meaning consistent with their meaning in the context of this specification and the relevant art and will not be interpreted in an idealized or overly formal sense unless expressly so defined herein. Further, terms such as distal, proximal, up, down, bottom, and top are relative, and are to aid illustration, but are not limiting. Relative terms such as "below" or "above" or "upper" or "lower" or "horizontal" or "vertical" may be used herein to describe a relationship of one element to another element as illustrated in the Figures. It will be understood that these terms and those discussed above are intended to encompass different orientations of the device in addition to the orientation depicted in the Figures. The embodiments are not intended to be mutually exclusive so that the features of one embodiment can be combined with other embodiments as long as they do not contradict each other. Terms of degree, such as "substantially", "about" and "approximately" are understood by those skilled in the art to refer to reasonable ranges around and including the given value and ranges outside the given value, for example, general tolerances associated with manufacturing, assembly, and use of the embodiments. The term "substantially" when referring to a structure or characteristic includes the characteristic that is mostly or entirely present in the structure. As used herein, the singular forms "a," "an," and "the" are intended to include the plural forms as well, unless the context clearly indicates otherwise. As used herein, the term "and/or" includes any and all combinations of one or more of the associated listed items. It will be understood that, although the terms first, second, etc., may be used herein to describe various elements, these elements should not be limited by these terms. These terms are only used to distinguish one element from another. For example, a first element could be termed a second element, and, similarly, a second element could be termed a first element without departing from the scope of the present disclosure. For simplification, the parts or elements of one embodiment which are found identically or similarly in the other embodiment will be identified using the same numerical references and will not be described again.

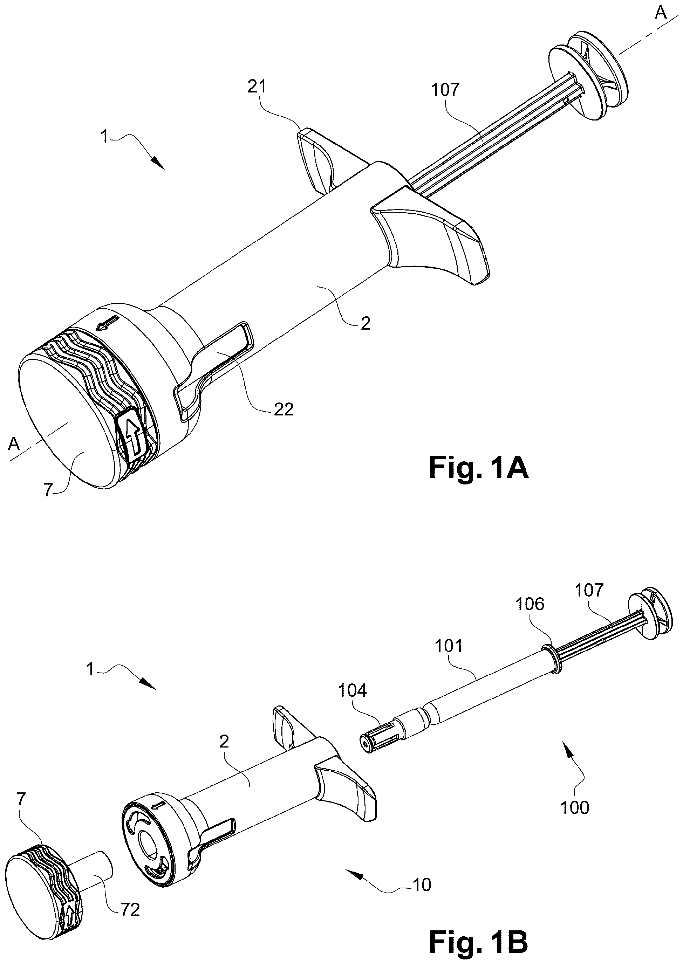

With reference to Figures 1A-1B is shown a manual injection device 1 according to an embodiment of the invention. The injection device 1 of the invention is configured to be grasped by a user such that the user can perform an injection operation. The injection device 1 1 includes a housing device 10 and a medical container 100 arranged within the housing 10.

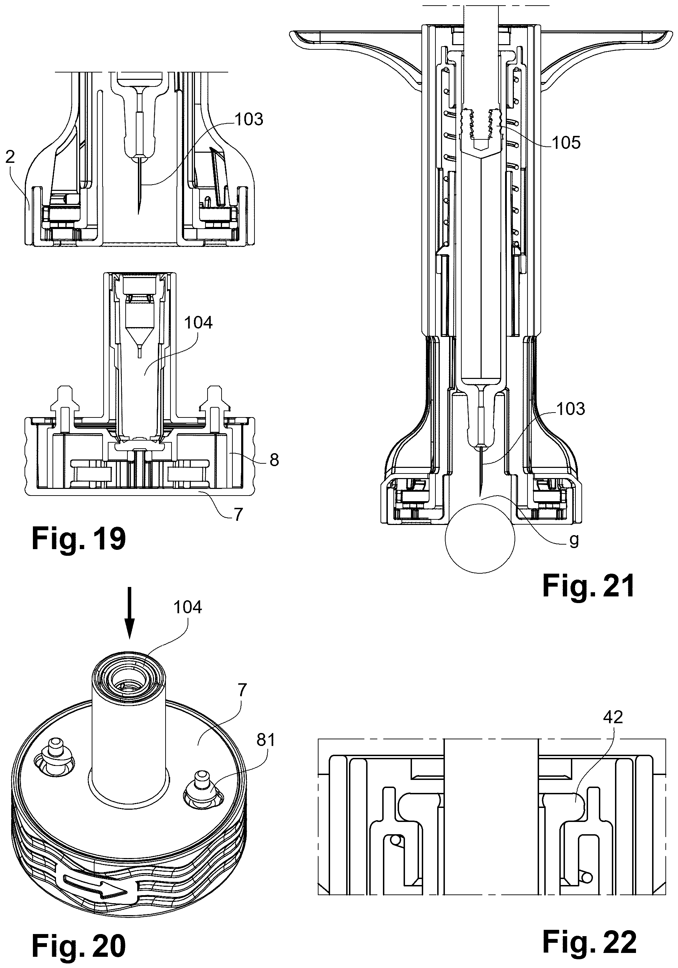

As illustrated in Figure 1B, the injection device 1 may include a medical container 100, such as a prefillable or prefilled syringe or a cartridge connected to a covered needle. The medical container 100 may include a tubular barrel 101 defining a reservoir configured for containing a medical product. The tubular barrel 101 may include an elongated distal tip 102 defining an axial passageway for passage of the medical product from the reservoir and an injection needle 103, Figure 21, attached to this distal tip 102. Prior to use of the injection device 1, the injection needle 103 is sealed and protected by a removable needle shield 104 removably attached to the distal tip 102. A stopper 105, Figure 21, is arranged within the barrel 101 for pushing fluid outside the barrel 101 via the distal tip 102 and the injection needle 103. At its proximal end, the barrel 101 may include a radial flange 106, such as a cut flange having a non-circular shape including two parallel edges or a round flange having a circular shape, which may serve to hold the medical container 100 at a fixed axial position within the housing 10. The barrel 101 may include or be made of a glass or a plastic material.

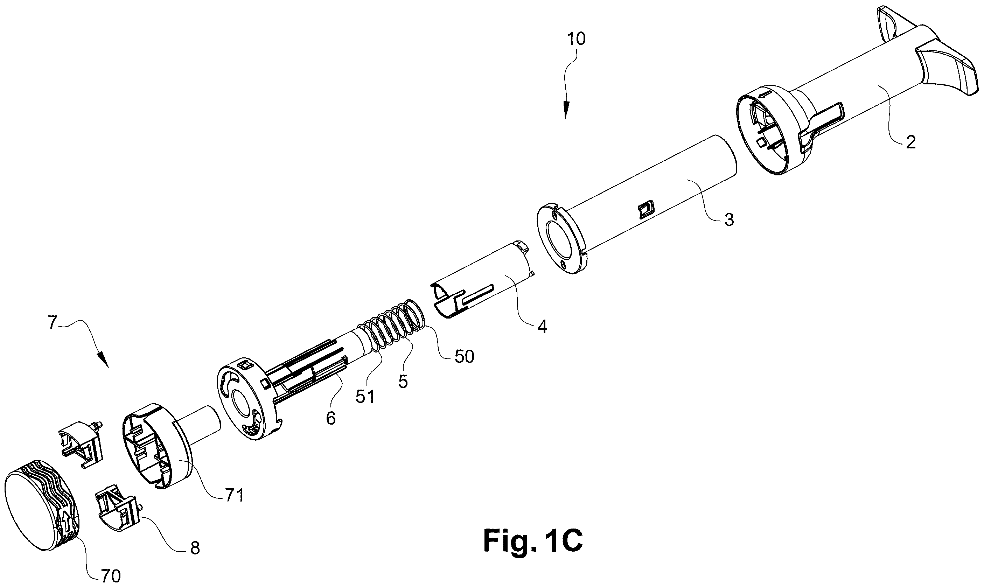

With reference to Figure 1C, the housing 10 includes some or all of: a shell 2, a locker 3, a holder 4, a safety spring 5, a body 6, a cap 7, and a reversible remover 8.

The shell 2 is configured for accommodating the locker 3, the holder 4, the safety spring 5, the body 6 and the medical container 100. The shell 2 is also configured to be grasped by the user. The shell 2 is further configured for guiding rotation of the locker 3.

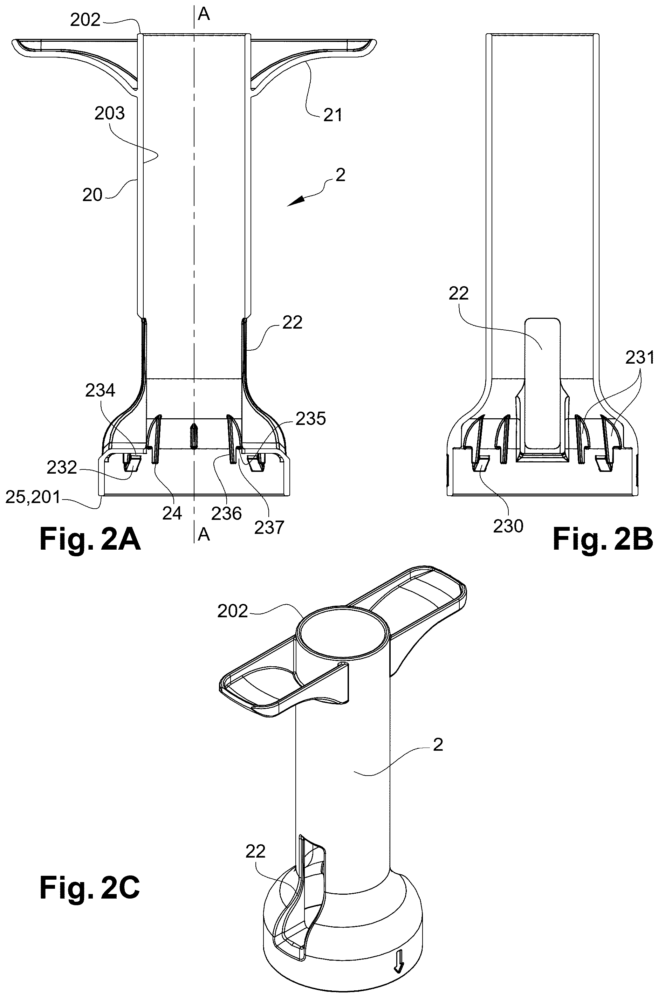

With reference to Figures 2A-2C, the shell 2 extends along a longitudinal axis A and includes a lateral wall 20 defining an inner cavity, an opened distal end 201 for inserting the locker 3, the holder 4, the safety spring 5, and the body 6 within the inner cavity, and an opened proximal end 202 for inserting the medical container 100 within the housing 10. The lateral wall 20 of the shell 2 has an inner surface 203 configured for guiding the locker 3 rotation.

The shell 2 further includes a finger flange 21 which may be arranged at the proximal end 202 of the shell 2 for allowing the user to position his/her fingers distal to the finger flange 21 on both sides of the shell 2. A see-through window 22 extends through the lateral wall of the shell 2 for allowing the user to see the container and the drug inside the shell 2 to inspect it. Securing means, such as one or more inner protrusions 230 and one or more axial fins 231, may be arranged at the distal end of the shell 2 for axially securing the body 6 with respect to the shell 2. The inner protrusion may have a distal ramp 232 for easing engagement of the body 6 and a proximal stop 234 for axially abutting against the body 6. The axial fins 231 may have a distal stop 235 for axially abutting against the body 6, and a rod 236 distally protruding from the distal stop 235, the rod 236 defining a notch 237 for accommodating a peripheral ring 604 of the body 6. The shell 2 may have a distal abutment 24 which may be arranged at the distal end of the shell 2, and more specifcally at a distal end of the rod 236, for axially abutting against the locker 3 such that the locker 3 is proximally blocked with respect to the shell 2, a distal seat 25 which may be delimited by the distal end 201 of the shell 2 for axially abutting against the cap 7.

The body 6 is configured for maintaining the locker 3 and the holder 4 within the shell 2. The body 6 is configured for closing the proximal end of the shell 2, while still allowing passage deployment of the injection needle 103 through the proximal end of the shell 2 to perform injection. The body 6 may further be configured to center the medical container 100.

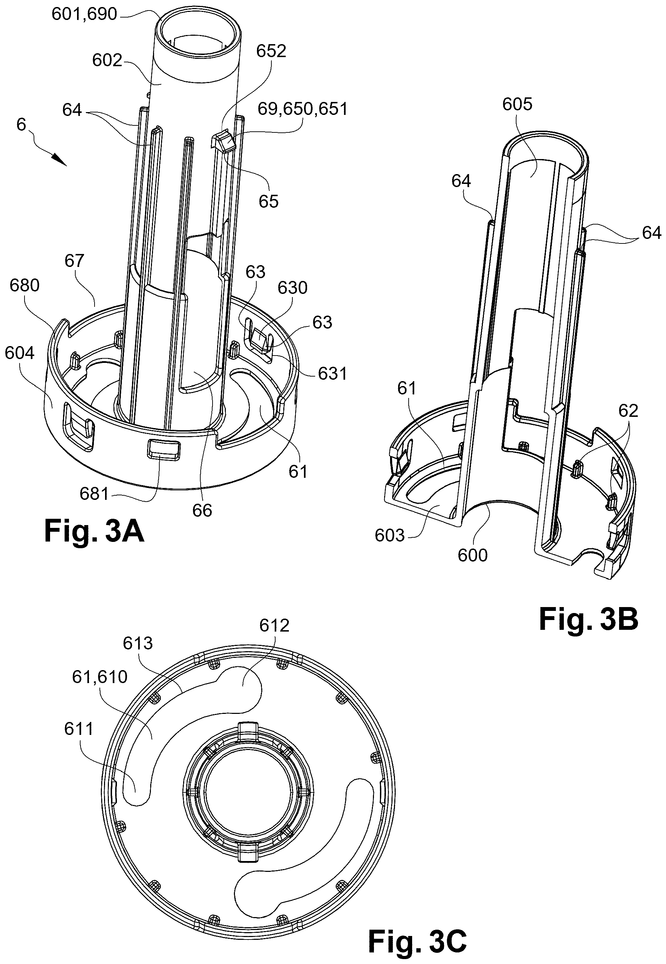

With reference to Figures 3A-3C, the body 6 may include an opened distal end 600 for allowing passage of the needle shield 104 and the injection needle 103 of the medical container 100, an opened proximal end 601 for allowing insertion of the medical container 100, a sidewall 602 defining an inner cavity for receiving the medical container 100, a functional flange 603 extending at the distal end 600 of the body 6, and a peripheral ring 604 proximally protruding at a periphery of the functional flange 603. The sidewall 602 has an inner surface 605 configured for axially guiding and centering the medical container 100.

The body 6 may include some or all of: a cam element 61, such as a circumferential slot 610 which may be provided through the functional flange 603, for causing movement of the reversible remover 8 in one way or the other. The circumferential slot 610 may include a locking end 611 shaped to forbid axial withdrawal of a pin 81 of a reversible remover 8, a releasing end 612 larger than the locking end 611 such that the releasing end 612 is shaped to allow axial insertion or extraction of said pin 81 through the functional flange 603, and a camming surface 613 which may converge towards the central longitudinal axis A from the locking end 611 to the releasing end 612 such that the pin 81 moving inside the circumferential slot 610 is pushed towards the central longitudinal axis A. The pin 81 may only be released when reaching the releasing end 612.

The body 6 may further include a proximal abutment 62, which may be arranged at a proximal end of short axial ribs extending on an inner surface of the peripheral ring 604, for axially abutting against the locker 3 such that the locker 3 is distally blocked with respect to the body 6. Orthoradial abutments 63 may be arranged on the peripheral ring 604 for orthoradially abutting against the locker 3 when the locker 3 is in its initial position and in its locking position. Therefore, the locker 3 is rotationally maintained in the initial position and in the locking position with respect to the body 6, unless a sufficient torque is applied to the locker 3, such as when the cap 7 is rotated by the user. The orthoradial abutments 63 may be arranged on both sides of a resilient tab 630 inwardly protruding from the peripheral ring 604. The resilient tab 630 may radially outwardly retract inside a window 631 of the peripheral ring 604 to allow rotation of the locker 3, and may radially inwardly deploy to engage a retaining member of the body 6, thereby retaining the locker 3 in the initial or locking position.

The body 6 may further include a proximal seat 64 which may be provided at a proximal end of axially extending outer ribs for providing support to a distal end 51 of the safety spring 5. A distal shoulder 65 may be arranged on the sleeve portion for distally abutting against the holder 4 such that the body 6 proximally blocks the holder 4 in its initial position. The distal shoulder 65 may be delimited at a distal end of an outer protrusion 650 outwardly radially protruding from the sidewall 602 to engage a guiding window of the holder 4, the protrusion 650 possibly including a proximal ramp 651 and a proximal abutment 652. A radial window 66 is arranged through the sidewall 602 for allowing the user to see the medical container 100 (the radial window 66 of the body 6 and the see-through window 22 of the shell 2 are radially aligned). The body 6 also includes a notch 67 arranged at a proximal end of the peripheral ring 604 for receiving a corresponding portion of the shell 2 including the see-through window 22. Securing means, such as a proximal end 680 of the peripheral ring 604 and openings 681 arranged through the peripheral ring 604 may be provided for axially and rotationally securing the body 6 to the shell 2. The poximal end 680 of the peripheral ring 604 may be configured for axially abutting against the proximal stop 234 of the shell 2, and the openings 681 may be shaped to complementarily receive the inner protrusions 230 of the shell 2. The body 6 is axially and rotationally fixed with respect to the shell 2.

The proximal abutment 62 of the body 6 and the distal abutment 24 of the shell 2 form securing means for axially securing the locker 3 with respect to the shell 2 and the housing 10.

In the illustrated embodiment, the body 6 and the shell 2 are two distinct parts, which are assembled to each other via the securing means.

The locker 3 is configured for axially locking the medical container 100 and the holder 4 within the housing 10.

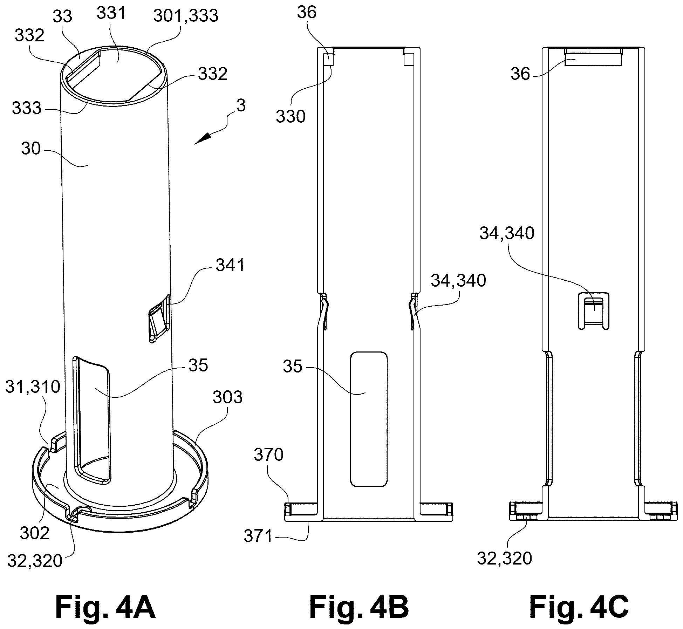

With reference to Figures 4A-4C, the locker 3 may include a tubular lateral wall 30 defining an inner cavity for receiving the body 6, a proximal end 301 delimiting a proximal opening for allowing passage of the medical container 100, a distal end delimiting a distal opening, a driving flange 302 which may extend around the distal end of the locker 3, and a peripheral rim 303 proximally protruding from a peripheral edge of the driving flange 302. The tubular lateral wall 30 of the locker 3 extends around the sidewall 602 of the body 6 while the peripheral ring 604 of the body 6 extends around the peripheral rim 303 of the locker 3.

The locker 3 may further include one or more retaining members 31, such as notches 310, arranged on the peripheral rim 303 for receiving the resilient tabs 630 of the body 6 and for orthoradially abutting against the body 6 in the initial release position and/or in the locking position such that rotation of the locker 3 from the release position to the locking position or vice versa is blocked by the body 6 until the user exerts a predetermined torque to the locker 3 via the cap 7. The locker 3 may include two pairs of diametrically opposite notches 310, arranged at 90° from each other. The locker 3 may include a driving member 32, such as radially extending slots 320 arranged through the driving flange 302 for cooperating with a corresponding driving member 32 of the cap 7, such as a pin 81 of a reversible remover 8, such that rotation of the cap 7 with respect to the shell 2 and the body 6 in a predetermined direction entails rotation of the locker 3 with respect to the shell 2 and the body 6 towards the locking position. The radially extending openings 320 are sized to allow radial translation of the reversible remover 8 from the initial position to the removing position. A locking member 33, such as distal shoulder 330 which may be arranged at the proximal end 301 of the locker 3, is provided for axially blocking the holder 4 and the medical container 100 within the housing 10 when the locker 3 is in the locking position. The locking member 33 may help delimit the proximal opening 331 of the locker 3, this opening 331 being complementarily shaped with the radial flange 106 of the medical container 100. For instance, the opening 331 has two straight edges 332 connected by circular portions 333 when the radial flange 106 is a so-called cut-flange.

A dampener 36, such as a soft part overmolded on the distal shoulder 330 may be arranged at a distal end of the locker 3 for limiting shocks to the medical container 100 when the cap 7 is being detached from the housing 10. The dampener 36 may be made of a soft flexible material such as thermoplastic elastomer (TPE), silicone, polyurethane (PU), although embodiments are not limited thereto.

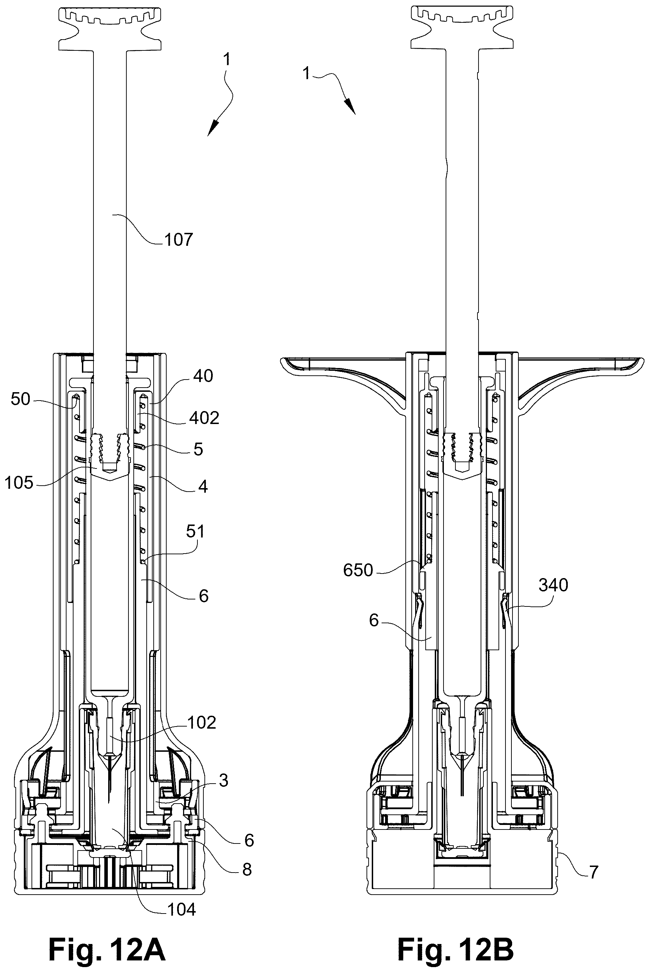

Back with reference to Figures 4B-4C and 12B, the locker 3 may alternatively or complementarily include a blocking element 34, here in the form of a resilient and proximally protruding leg 340, arranged through a window 341 of the tubular lateral wall 30 for temporarily preventing axial movement of the holder 4 and the medical container 100 with respect to the body 6.

A side window 35 provided through the tubular lateral wall 30 for allowing the user to see the medical container 100 when (and only when) this side window 35 is radially aligned with the radial window 66 of the body 6 and the see-through window 22 of the shell 2 (i.e. when the locker 3 has been rotated in the locking position). The locker 3 also comprises securing means, such as a proximal end 370 of the peripheral rim 303 and a distal side 371 of the driving flange 302, for axially securing the locker 3 with respect to the housing 10. The proximal end 370 of the peripheral rim 303 is configured for axially abutting against the distal abutment 24 of the shell 2, while the distal side 371 of the driving flange 302 is configured for axially abutting against the proximal abutment 62 of the body 6.

The locker 3 may be axially fixed with respect to the housing 10, but is rotationally movable around the longitudinal axis A with respect to the housing 10 and/or the holder 4 between an initial release position in which the locker 3 allows for insertion or removal of the medical container 100 within the housing 10, and a locking position in which the locker 3 blocks the medical container 100 within the housing 10 thereby preventing removal of the medical container 100 from the housing 10. Rotation of the locker 3 from the release position to the locking position is caused by the cap 7 being rotated by the user.

The holder 4 is configured for receiving and holding the medical container 100.

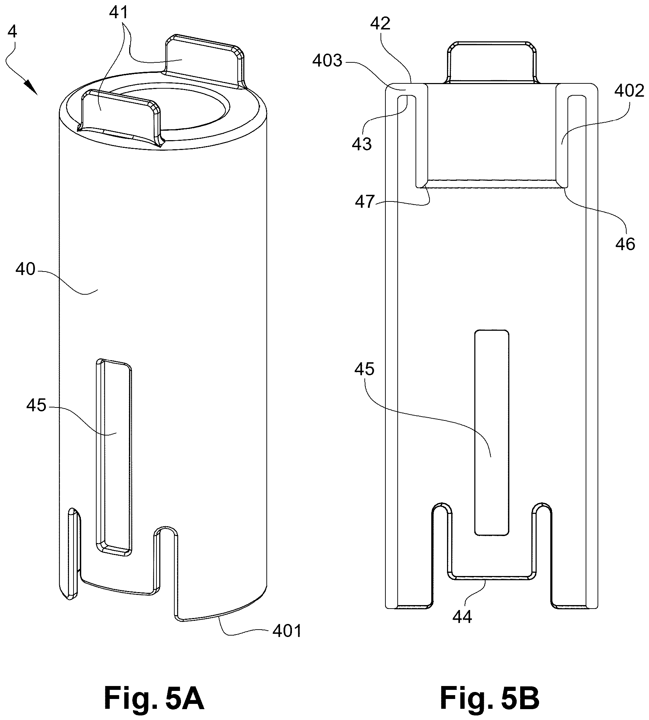

With reference to Figures 5A-5B, the holder 4 may include a cylindrical lateral wall 40 for receiving the medical container 100, a proximal end delimiting a proximal opening, a distal end 401 delimiting a distal opening, an inner ring 402 for receiving the barrel 101 of the medical container 100, and a collar 403 connecting the inner ring 402 to the cylindrical lateral wall. The inner ring 402 may extend at the proximal end of the holder 4.

The holder 4 may further include one or more radial retainers 41 such as axial tabs proximally protruding from the proximal end of the holder 4 or from a proximal side of the collar 403 for radially abutting against the radial flange 106 of the medical container 100. A proximal abutment 42 may be defined by a proximal side of the collar 403 or at the proximal end of the holder 4 for axially abutting against a distal side of the radial flange 106, thereby stopping insertion of the medical container 100 inside the housing 10. The proximal abutment 42 (together with the distal shoulder 65 of the body 6) also helps define the axial position of the medical container 100 within the housing 10 such that, when the needle shield 104 has been removed from the medical container 100, Figure 21, an axial gap g separates a distal end of the injection needle 103 from a theoritecal ball having a predetermined diameter of for instance 12 mm. This provides safety for the user. The holder 4 further has a distal seat 43 which may be delimited by a distal side of the collar 403 for providing support to the proximal end 50 of the safety spring 5. A distal blocking surface 44 may be arranged at a distal end of a distal leg for axially abutting against the blocking element 34 of the locker 3, thereby temporarily maintaining the holder 4 in its initial position until insertion of the injection needle 103 in the injection site. Axial notches may extend on both sides of the distal leg to ease assembly of the holder 4 and the body 6. The holder 4 also includes a guiding member 45 such as a guiding window extending through the cylindrical lateral wall of the holder 4 for receiving a guiding member 69 of the body 6, such as a radial hook defining the distal shoulder of the body 6, thereby axially guiding movement of the holder 4 with respect to the body 6 while preventing rotation of the holder 4 around the longitudinal axis A with respect to the body 6. An injection depth controlling member 46, such as a distal abutment surface, may be arranged at a distal end of the inner ring 402 for axially abutting against a proximal stop 690 which may be arranged at the proximal end 601 of the body 6, such that distal movement of the holder 4 (and thus of the medical container 100) is blocked at a predetermined axial position to control the injection depth. The holder 4 may include a proximally tapered surface 47, such as a chamfer arranged at a distal end of the inner ring 402 for avoiding that the needle shield 104 clings to the holder 4 when the used medical container 100 is withdrawn from the housing 10.

The holder 4 is axially movable with respect to the body 6 along the longitudinal axis A between an initial position in which the safety spring 5 is expanded and the distal shoulder of the body 6 axially abuts against the holder 4, and in which the bocking element 34 of the locker 3 axially abuts against the holder 4, and an injection position, distally located with respect to the initial position, in which the safety spring 5 is compressed so that the medical container 100 is moved distally and pricks the injection site at the proper injection depth to then expel the medical product. Movement of the holder 4 from the initial position to the injection position may be caused by the user pressing against the plunger rod 107 of the medical container 100 in the distal direction direction. The friction force between the stopper 105 and the barrel 101 overcomes the resistance of the blocking element 34 of the locker 3 such that the holder 4 moves to the injection position, i.e. injection needle 103 pricked in the targeted tissues, together with the medical container 100 while the plunger rod 107 has not yet begun to move the stopper 105 within the barrel 101. The holder 4 may be rotationally fixed with respect to the body 6.

As visible in Figure 12A, the safety spring 5 is arranged between the holder 4 and the body 6 for maintaining the holder 4 in the initial position. The safety spring 5 may be a coil spring and may include a proximal end 50 arranged between the inner ring 402 and the cylindrical lateral wall 40 of the holder 4 and a distal end 51 extending around a proximal portion of the body 6.

The cap 7 is configured for closing the distal end of the housing 10 and for removing or repositioning the needle shield 104 from or onto the injection needle 103 of the medical container 100.

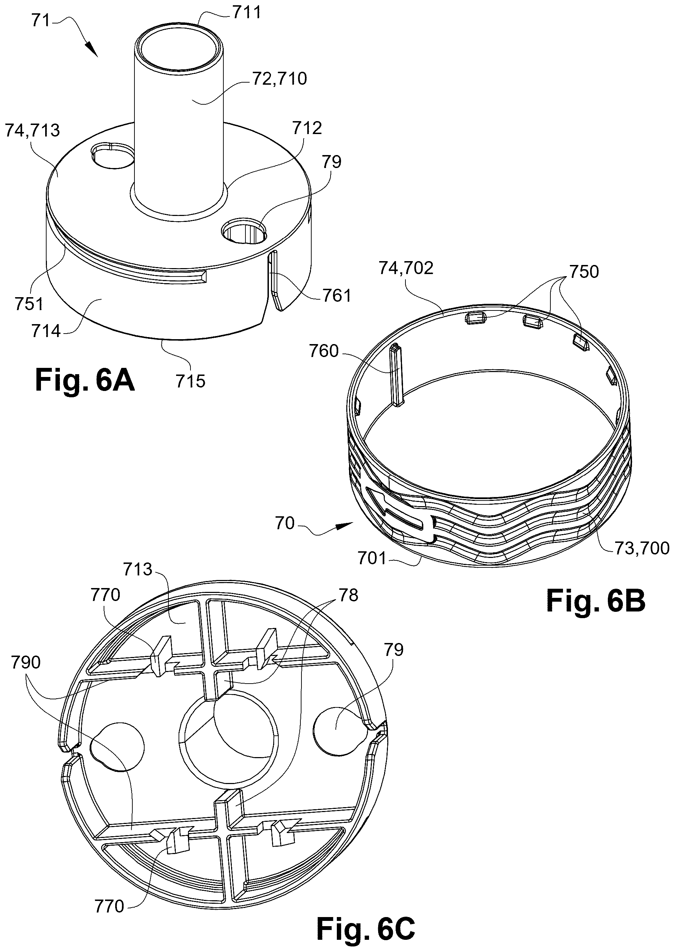

With reference to Figures 6A-6B, the cap 7 may be made of a first part 70 and a second part 71 assembled to each other, the first part 70 and the second part 71 delimiting an inner cavity accommodating a reversible remover or gripper 8 which will be described in further details below. The first part 70 may have a lateral wall 700, a closed distal end 701 and an opened proximal end 702. The second part 71 may have a sleeve member 710 defining an inner cavity for receiving the needle shield 104 of the medical container 100, the sleeve member 710 having an opened proximal end 711 and an opened distal end 712, an outer flange 713 which may be arranged at the distal end 712 of the sleeve member 710 for closing the proximal end 702 of the first part 70, and a distal skirt 714 distally protruding from a peripheral edge of the outer flange 713, the distal skirt 714 delimiting an opened distal end 715.

The cap 7 may further include a guiding member 72, which may be an outer surface of the sleeve member 710, for guiding and centering the cap 7 within the body 6. The guiding member 72 is also configured for protecting the user by preventing the user from touching or picking the needle shield 104. This also avoids losing the needle shield 104. The guiding member 72 may have a cylindrical or hemi-cylindrical shape.

The cap 7 may further include a grasping surface 73 which may be an outer surface of the lateral wall 700 of the first part 70 for allowing the user to grasp and rotate the cap 7 with respect to the housing 10, a proximal abutment 74 which may be arranged at the proximal end 702 of the first part 70 or at a proximal side of the outer flange 713 for axially abutting against the housing 10, thereby stopping insertion of the cap 7 into the housing 10. The cap 7 also includes securing means, such as radial inner protrusions 750 which may be arranged on the first part 70 for complementarily engaging a tangential slot 751 of the second part 71, thereby axially and/or rotationally securing the first part 70 and the second part 71, and may include indexing means, such as an axial rib 760 which may be arranged on the first part 70 and an axial slot 761 which may be arranged on the second part 71, for positioning the first part 70 and the second part 71 at a predetermined relative angular position allowing their assembly. The cap 7 may further include one or more snap-fit features, such as axial tabs 770 distally protruding from the outer flange 713 inside the second part 71 for maintaining the reversible remover 8 in a locking position, one or more radial stops 78 which may be delimited by radial ribs extending within the second part 71 for radially abutting against the reversible remover 8, one or more openings 79 provided through the outer flange 713 for allowing extension of a cam member 85 of the reversible remover 8 therethrough, and one or more guiding walls 790 for guiding movement of the reversible remover 8 in a radial direction. An opening 791 may be arranged between the axial tab 770 and the guiding walls 790 for receiving the snap-fit feature 86 of the reversible remover 8 when the reversible remover 8 is in the release position. This opening avoids creeping of the snap-fit feature 86 of the reversible remover 8 that could be caused by a long storage period.

The cap 7 is removable from the housing 10. The cap 7 is rotatable around the longitudinal axis A with respect to the housing 10 between an initial position, in which the cap 7 may be axially locked to the housing 10, and a release position, in which the cap 7 can be axially detached from the housing 10. Movement of the cap 7 from the initial position to the release position is caused by the user grasping the cap 7 and turning it in the appropriate rotational direction.

In the illustrated embodiment, the cap 7 is made of two distinct parts. However, embodiments are not limited thereto and the cap 7 could alternatively be made of a single piece.

The reversible remover 8 is configured for removing the needle shield 104 of the medical container 100 before injection and for releasing the needle shield 104 so that the needle shield 104 can be re-attached to the used medical container 100 after injection. By reversible remover 8 it is meant that the remover 8 can capture the needle shield 104 but also subsequently release the needle shield 104 when re-positioning the cap 7 on the body 6 and rotating the cap 7 back from the release to the locking position.

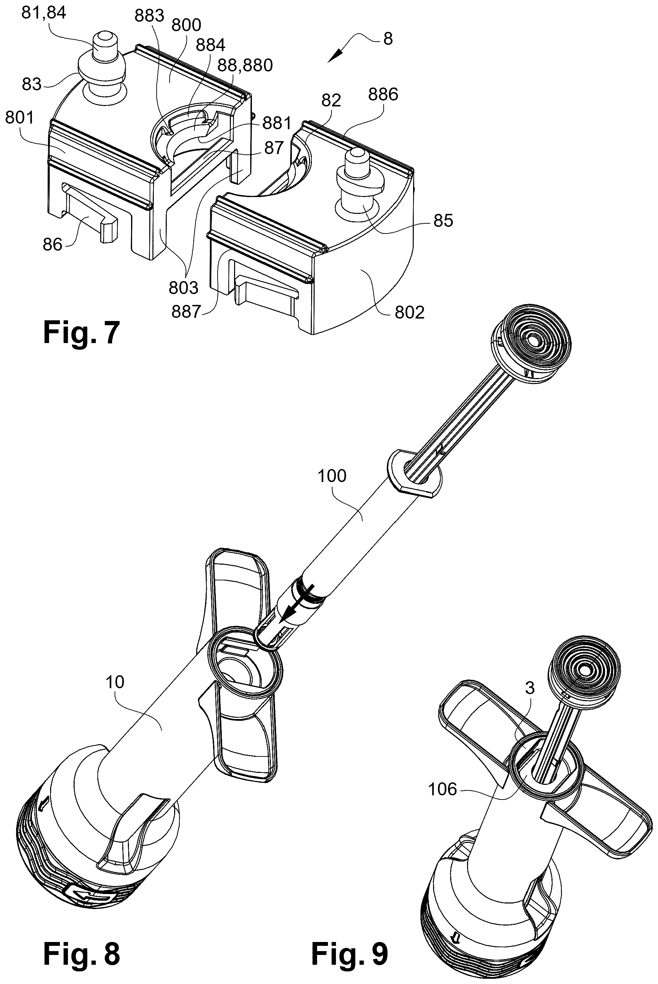

With reference to Figure 7, the reversible remover 8 may include a proximal side surface 800, two parallel lateral surfaces 801, a partially cylindrical outer lateral surface 802, four distally protruding legs 803 so that the reversible remover 8 can rest on the closed distal end 701 of the first part 70 of the cap 7, and a pin 81 proximally protruding from the proximal side surface 800. Opposite the partially cylindrical outer lateral wall 802, the reversible remover 8 may include a semi-circular opening 82 for receiving the needle shield 104 of the medical container 100.

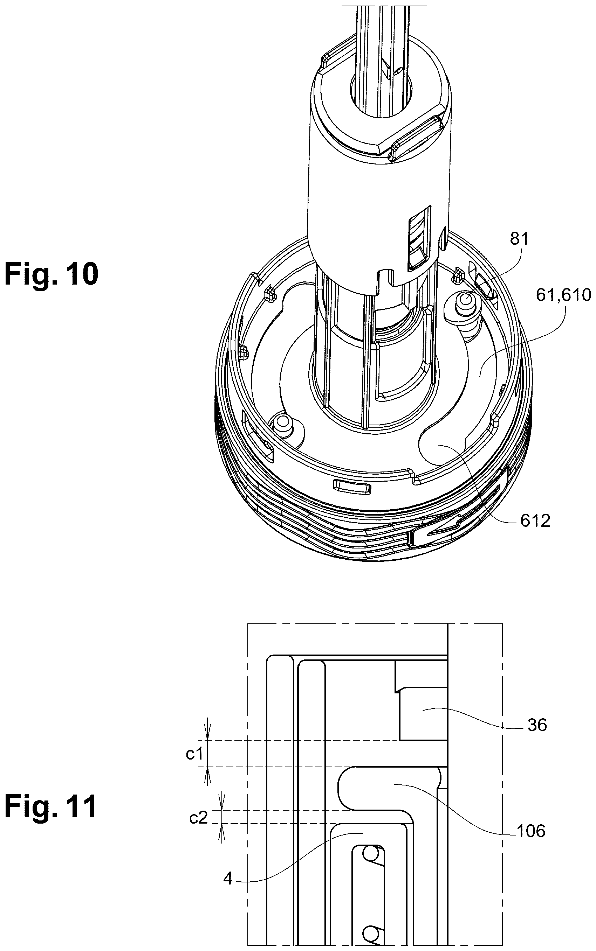

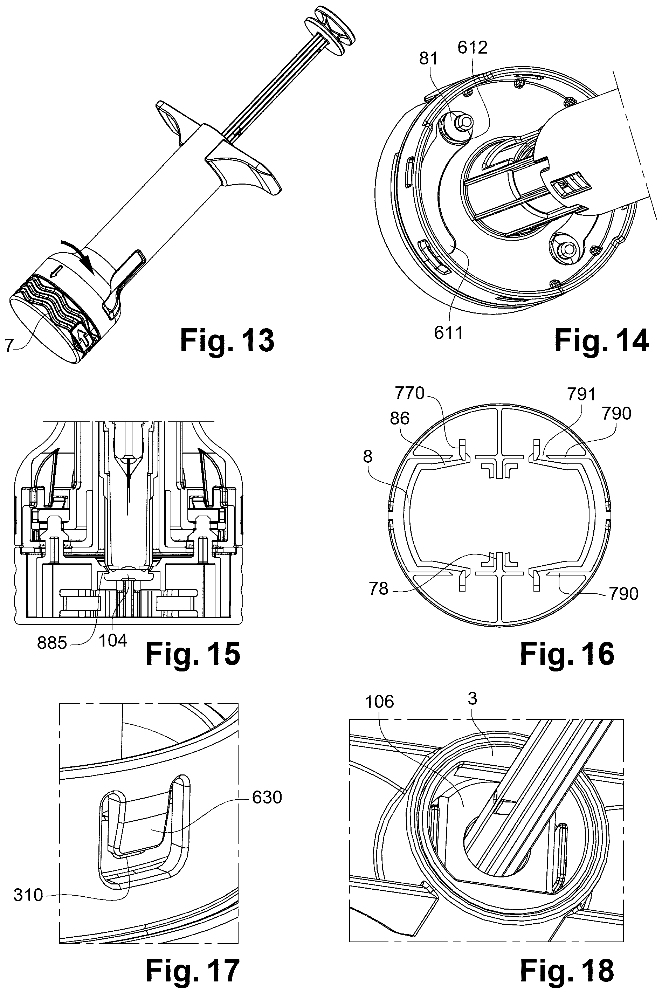

Still with reference to Figure 7, the reversible remover 8 may further include a locking element 83, such as a distal abutment formed at a distal side of a collar radially extending around the pin 81, for axially abutting against a proximal side of the functional flange 603 of the body 6 in order to prevent removal of the cap 7 as long as the cap 7 is in the initial position. A driving member 32 may be arranged at a distal end of the pin 81 for causing rotation of the locker 3 when the cap 7 is rotated with respect to the housing 10. That is, rotation of the cap 7 from the initial position to the removing position may cause simultaneous rotation of the locker 3 from the initial position to the locking position. The reversible remover 8 includes a cam member 85 which may be arranged between the locking element 83 and the proximal side surface 800 and which engages the cam member 61 of the body 6 such that rotation of the cap 7 causes radial movement of the reversible remover 8 from a release position to a locking position and vice versa. A snap-fit feature 86, such as a resilient arm, may be arranged at a side of the reversible remover 8, and may for instance radially protrude from one of the distally protruding legs 803, for engaging the corresponding snap-fit feature 770 of the cap 7 in order to maintain the reversible remover 8 in the locking position. The reversible remover 8 has a proximal stop 87, for instance at proximal side of a strut, for axially abutting against a distal end of the needle shield 104 of the medical container 100, thereby stopping insertion of the medical container 100 within the housing 10. The proximal stop 87 may be configured such that there are small axial clearances c1, c2, Figure 11, on both sides of the radial flange 106 of the medical container 100 before removal of the needle shield 104. That is, before removal of the needle shield 104, the radial flange 106 is axially away from the locking member 33 of the locker 3 and the proximal abutment 42 of the holder 4. Thus, the axial position of the medical container 100 and of the needle shield 104 is accurately controlled ; this ensures the future tightening of the needle shield 104 by the reversible removers 8. A removing member 88, such as a semi-circular rib 880 including a sharp edge 881 for engaging either the needle shield 104, may be extend at the semi-circular opening 82 and may be connected to the proximal side surface 800 by bridges 883 delimiting slots 884 therebetween in order to capture the needle shield 104. The removing member 88 defines a distal abtument 885, Figure 15, for exerting a distal force on the needle shield 104 when the cap 7 is axially removed from the housing 10. Proximal sliding surfaces 886, which may be formed at a crest of two parallel ribs proximally protruding from the proximal side surface, may be provided for axially abutting against the distal side of the outer flange 713 of the cap 7 while limiting friction between the reversible remover 8 and the cap 7 when the reversible remover 8 moves from the release position to the removing position or vice versa. Likewise, lateral sliding surfaces 887, which may be formed at a crest of two ribs protruding from the lateral surfaces 801, may be provided for axially abutting against the distal side of the outer flange 713 of the cap 7 while limiting friction between the reversible remover 8 and the cap 7 when the reversible remover 8 moves from the release position to the removing position or vice versa.

The remover 8 is radially movable between a release position, radially outwardly located with respect to the needle shield 104 and the central longitudinal axis A, in which the reversible remover 8 does not engage the needle shield 104, and a removing position, radially inwardly located with respect to the release position, in which the reversible remover 8 engages the needle shield 104 such that axial withdrawal of the cap 7 from the housing 10 entails removal of the needle shield 104 from the medical container 100. Movement of the reversible remover 8 from the release position to the removing position, or vice versa, is caused by the cap 7 rotating around the longitudinal axis A together with the reversible remover 8 with respect to the body 6 and to the radial abutment between the cam member 85 of the reversible remover 8 and the cam element 61 of the body 6. The remover 8 may be axially fixed with respect to the cap 7.

The injection device 1 may include one or more, for instance two diametrically opposite, reversible removers 8 which act on both sides of the needle shield 104. The reversible remover 8 may include a resiliently deformable material, such as for instance polyoxymethylene (POM), polypropylene (PP), or acrylonitrile butadiene styrene (ABS), although embodiments are not limited thereto. The reversible remover 8 may be made of a single piece.

Operation of the injection device 1 according to an embodiment of the invention is described below in connection with Figures 8-33.

Initially, the cap 7 is attached to the housing 10 and the reversible remover 8 is in the release position. The pin 81 of the reversible remover 8 is engaged in the locking end 611 of the circumferential slot 610 of the body 6 such that the cap 7 cannot be detached from the housing 10, Figure 10.

The user first places a new medical container 100 within the housing 10, as illustrated in Figure 8. The locker 3 is in the initial position and in this position, the tubular lateral wall 30 of the locker 3 closes the see-through window 22 of the shell 2 such that the user cannot see inside the housing 10 before injection. The medical container 100 may be arranged in a predetermined angular position with respect to the housing 10 due to the shape of the proximal end of the locker 3, as visible on Figure 9.

Insertion of the medical container 100 is stopped by the proximal stop 87 of the reversible remover 8. In this position, the radial flange 106 of the medical container 100 is separated from the holder 4 by a first clearance c1, Figure 11, and from the locker 3 by a second clearance c2, which will allow for a reliable clamping of the needle shield 104 by the reversible remover 8.

In order to remove the cap 7, the user first needs to rotate the cap 7 with respect to the housing 10, Figure 13. As a consequence, the pin of the reversible remover 8 travels along the circumferential slot 610 of the body 6, from the locking end 611 to the release end 612, Figure 14. By doing so, the pin 81 slides against the camming surface 613 of the circumferential slot 610, which causes the reversible remover 8 to simultaneously translates radially inwardly. Accordingly, the removing member 88 reaches the removing position, i.e. engages the needle shield 104, Figures 15-16. The snap-fit features 86 of the reversible remover 8 are engaged with the cap 7, thereby retaining the reversible remover 8 in the removing position. Besides, since the pin 81 is in the release end, the cap 7 is no longer locked to the body 6 : the user can pull the cap 7 to remove it from the housing 10, Figure 19. Since the reversible remover 8 is engaged with the needle shield 104, removal of the cap 7 entails simultaneous removal of the needle shield 104, thus unveiling the injection needle 103.

Due to the abutment between the pin 81 and the locker 3, the locker 3 has also rotated, for instance 90°, from the initial position to the locking position, Figure 18, such that the locking member 33 of the locker 3 now faces and bars the radial flange 106 of the medical container 100 from moving outside the housing 10. Moreover, the window of the locker 3 is now aligned with the see-through window 22 of the shell 2.

During removal of the cap 7, the medical container 100 is pressed against the holder 4, but does not move distally further since the holder 4 is axially blocked by the blocking element of the locker 3, Figure 22. When the needle shield 104 is detached from the medical container 100, this medical container 100 tends to move proximally and may hit the locker 3. The dampener 36 allows for limiting the impact between the medical container 100 and the locker 3.

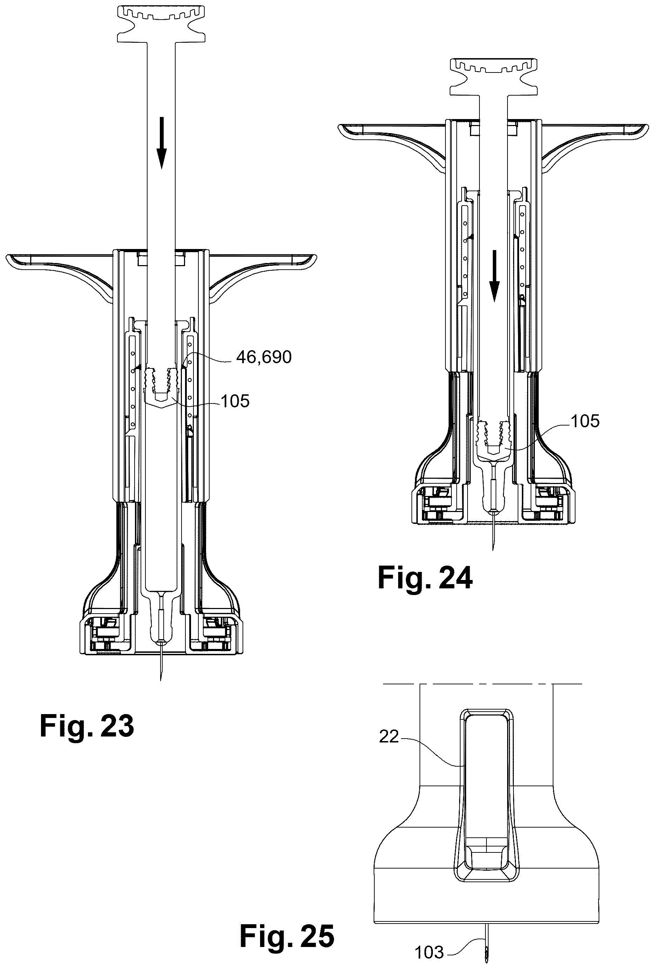

The injection device 1 is ready for injection. As a safety measure, the injection needle 103 stays inside the housing 10, Figure 21, thereby avoiding needlestick injuries. The user positions the housing 10 against an injection site and pushes the plunger rod 107 in the distal direction. The distal force exerted on the plunger rod 107 overcomes the blocking action of the locker 3 : the blocking element 34 radially outwardly retracts so that the holder 4 can move in the distal direction, thereby compresssing the safety spring 5. The medical container 100 can move together with the holder 4 in the distal direction too. As a result, the injection needle extends distally beyond the distal end of the housing 10 and penetrates into the injection site, until the holder 4 abuts against the body 6. The injection needle 103 reaches the proper injection depth, Figure 23.

The user goes on exerting a distal force upon the plunger rod 107. Consequently, the plunger rod 107 overcomes the friction force between the stopper 105 and the barrel 101, and pushes the stopper 105 in the distal direction, thereby expelling the medical product into the injection site, until the stopper 105 abuts against the distal end of the reservoir formed by the barrel 101, Figure 24. Thanks to the see-through window 22 and the window of the locker 3, which are aligned, the user can see the end of injection.

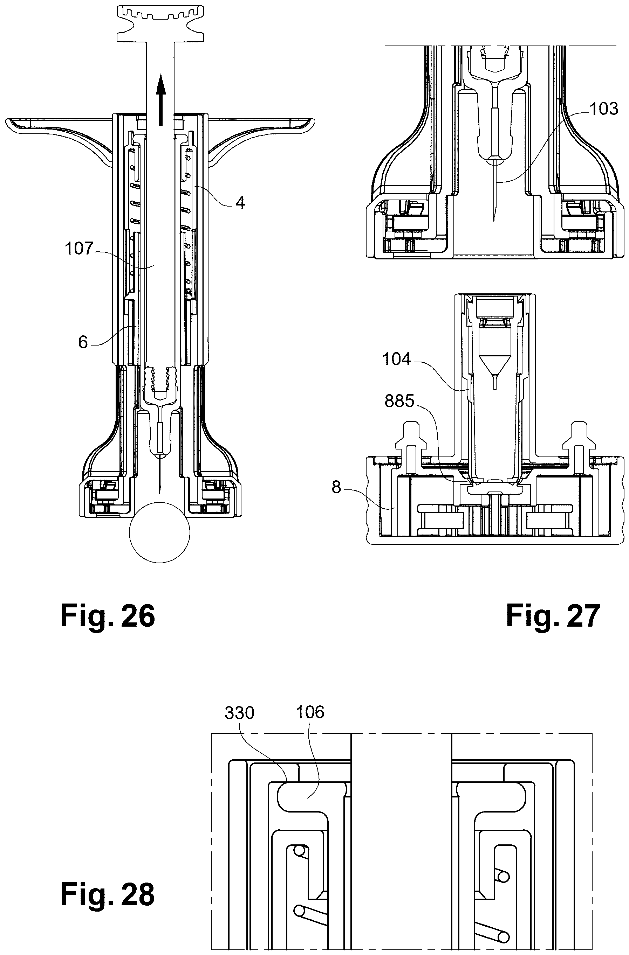

When the user releases the pression on the plunger rod 107, the safety spring 5 moves expands, thus moving the holder 4 together with the medical container 100 in the proximal direction. The injection needle 103 accordingly retracts within the housing 10, Figure 26.

The user can then re-attach the cap 7 to the housing 10. This requires the pin 81 to be axially aligned with the release end 612 of the cam element 61. By doing so, the captured needle shield 104 is also put back onto the medical container 100, Figure 27, covering again the injection needle 103. The medical container 100 is pushed in the proximal direction, but the locker 3 blocks the medical container 100, such that the needle shield 104 can be repositioned on the used medical container 100.

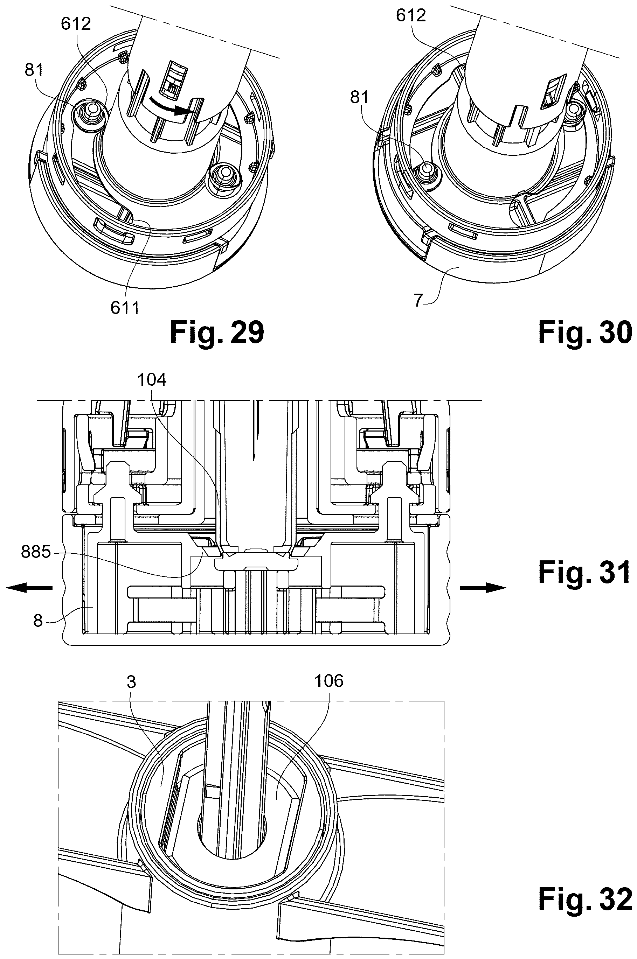

The user can then rotate the cap 7 back by a 90° rotation, Figure 29. As a consequence, the pin 81 of the reversible remover 8 moves along the track 610 of the body 6, from the release end 612 to the locking end 611. The cap 7 gets locked again to the body 6. Besides, the reversible remover 8 moves back in the radial direction towards the release position, Figure 31, such that the needle shield 104, which has been put back onto the medical container 100, is disengaged. The locker 3 also turns back towards the initial position, and thus no longer blocks the medical container 100 within the housing 10, Figure 32. The injection device 1 is reset.

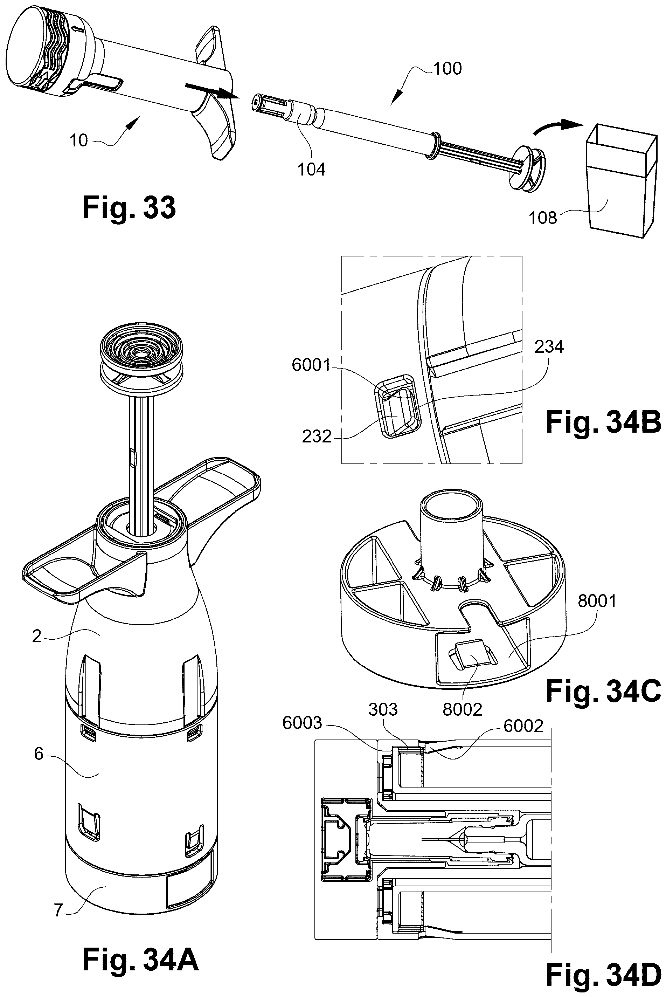

The medical container 100 can then be disposed in a sharps container 108, Figure 33. The injection device 1, which has been reset, can now be used in association with a new medical container 100.

The device of the invention can thus be re-used instead of being discarded together with the used medical container 100. This improves sustainability. This re-usable feature of the device of the invention is realized without any complexification, i.e. while having the advantage of being simple to manufacture, assemble and use.

Figures 34A to 46C illustrate the injection device 1 according to other embodiments. For simplification, the parts or elements of one embodiment which are found identically or similarly in the other embodiment will be identified using the same numerical references and will not be described again. It is reminded that the embodiments are not intended to be mutually exclusive so that the features of one embodiment can be combined with other embodiments as long as they do not contradict each other. Unless otherwise specified, operation of the below-described devices is similar to the one described in connection with Figures 1A-33.

Figures 34A to 34D illustrate the injection device 1 according to another embodiment. Here, the body 6 does not extend within the shell 2, but distal to the shell 2. The securing means for securing the body 6 to the shell 2 here include the proximal stop 234 on a proximal side of a resilient tab whose distal side has the distal ramp 232, for engaging an opening arranged on a proximal end 601 of the body 6, the opening defining a distal stop 6001 for axially abutting against the proximal stop 234 of the shell 2.

Besides, the cap 7 is here made of a single part, having a radial opening 8001 for allowing insertion of the reversible remover 8. The snap-fit feature is here in the form of resilient tongues 8002 arranged on the closed distal end 712 of the cap 7 for retaining the reversible remover 8 in the removing position.

The locker 3 is here axially blocked by the body 6: the body 6 has a resilient leg defining a distal abutment 6002 and the body 6 further has a proximal abutment 6003, such that the peripheral rim of the locker 3 is sandwiched between the distal abutment 6002 and the proximal abutment 6003 of the body 6.

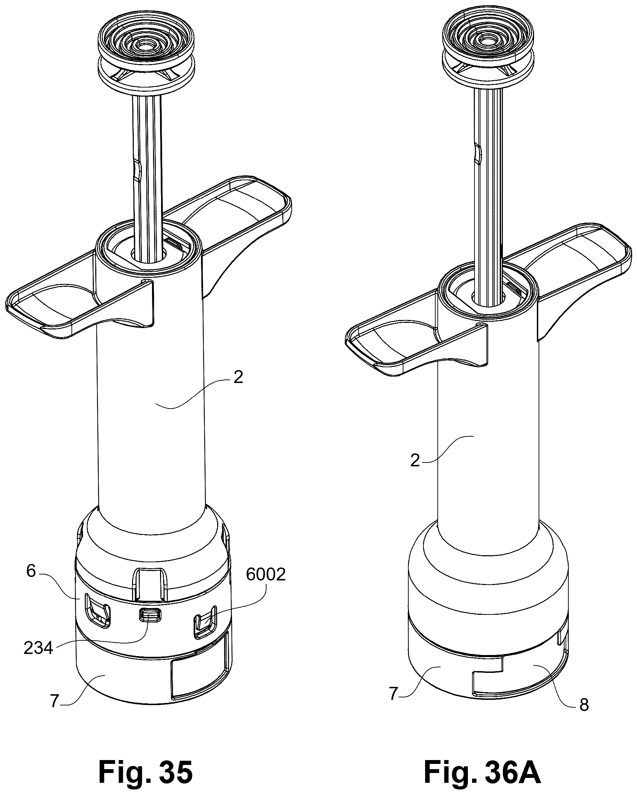

Figure 35 illustrates the injection device 1 according to another embodiment. This embodiment is similar to the one illustrated in Figures 34A-34D, except that the shape and dimensions of the shell 2 and the body 6 are different. Specifically, the shell 2 here has a smaller diameter and the body 6 is axially shorter than in the previously described embodiment. Varying the shape, length, and/or diameter of the shell 2 and/or the body 6 enables to ease grasping of the injection device 1 for specific users.

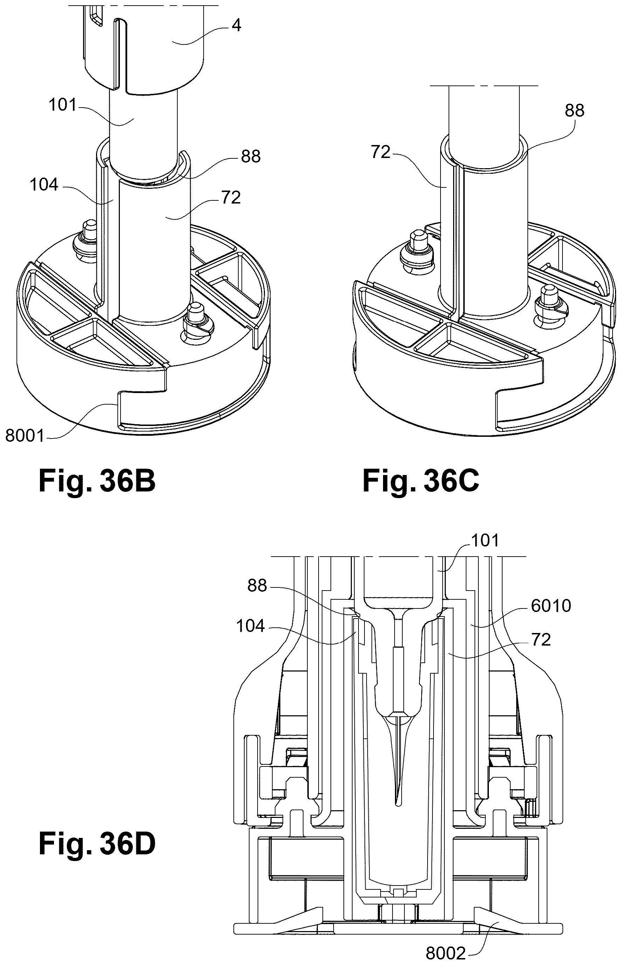

Figures 36A to 36D illustrate the injection device 1 according to another embodiment. Instead of engaging a distal end of the needle shield 104, the reversible remover 8 is here configured for engaging the proximal end of the needle shield 104, and more specifically to extend within an axial gap separating the proximal end of the needle shield 104 and a distal shoulder of the barrel 101 of the medical container 100. To that end, the cap 7 is devoid of the guiding member 72. Instead, the guiding member 72 is arranged on the reversible remover 8 and is configured to surround the needle shield 104 such that the removing member 88 arranged at the proximal end of the guiding member 72 engages the gap between the barrel 101 and the needle shield 104. The cap 7 may include the resilient tongues 8002 for retaining the reversible remover 8 in the removing position. The body 6 now includes an enlarged distal cavity 6010 for accommodating the guiding member 72, when the reversible remover 8 is in the release position, Figure 36D. When the cap 7 is rotated, the reversible remover 8 moves radially inwardly such that the removing members 88 engage between the proximal end of the needle shield 104 and the barrel 101, Figure 36D.

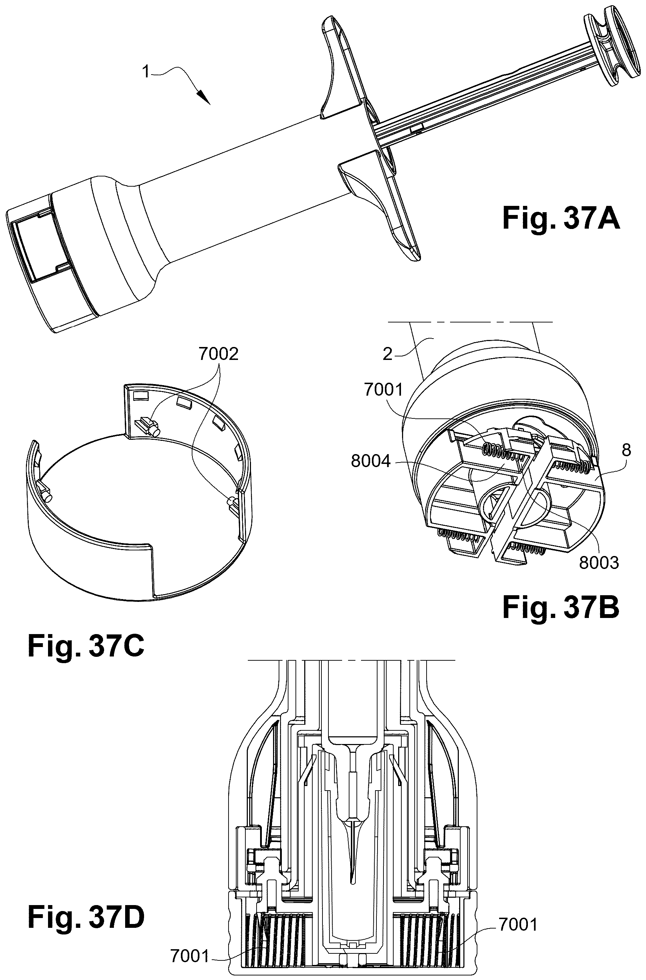

Figures 37A to 37D illustrate the injection device 1 according to another embodiment. The cap 7 now includes biasing means, such as one or more, for instance four, springs 7001, configured to help move the reversible remover 8 towards the removing position. The springs extend between a pin 7002 of the cap 7 and a radial seat 8003 defined by a recess 8004 of the reversible remover 8. These compression springs thus improve the hold of the removing member 88 on the needle shield 104, thereby improving reliability of the needle shield 104 removal. The springs and the reversible remover 8 may be distinct parts. Alternatively, Figure 37D, the cap 7 may include only two springs 7001.

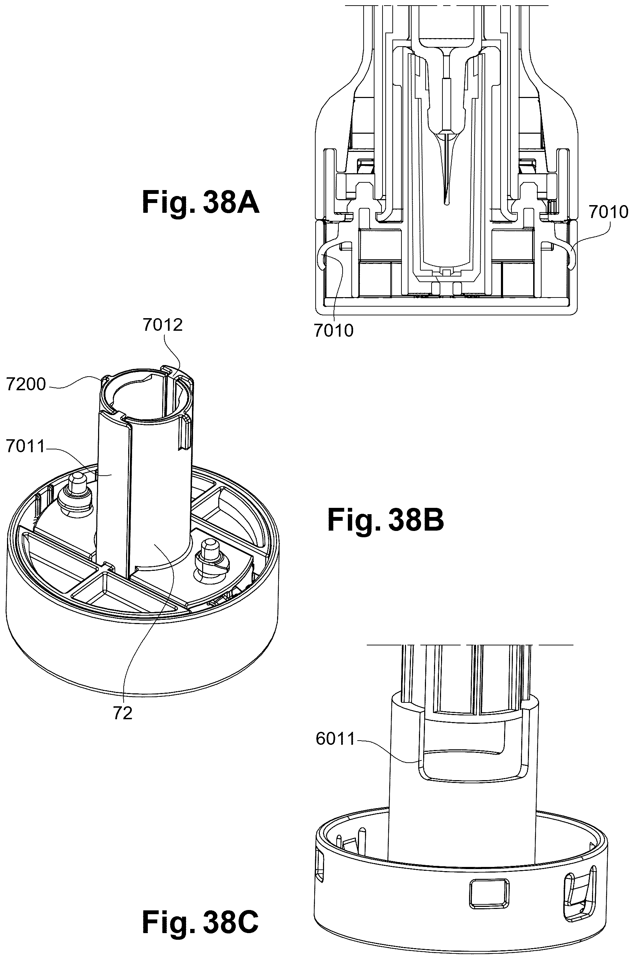

Figures 38A to 38C illustrate the injection device 1 according to another embodiment. The biasing means here are formed by one or more, for instance two spring blades 7010 which are configured to move the reversible removers 8 towards the removing position. The spring blade 7010 and the corresponding reversible remover 8 may be made of a single piece. Besides, the guiding member 72 may now include an axial rib 7200 configured for cooperating with a complementarily shaped tangential groove 6011 of the body 6, and the cap 7 may include two diametrically opposite and distally protruding legs 7011 for guiding reinsertion of the cap 7 within the body 6. The legs 7011 may include an inner axial rib 7012 extending between the removing members 88 of the reversible remover 8.

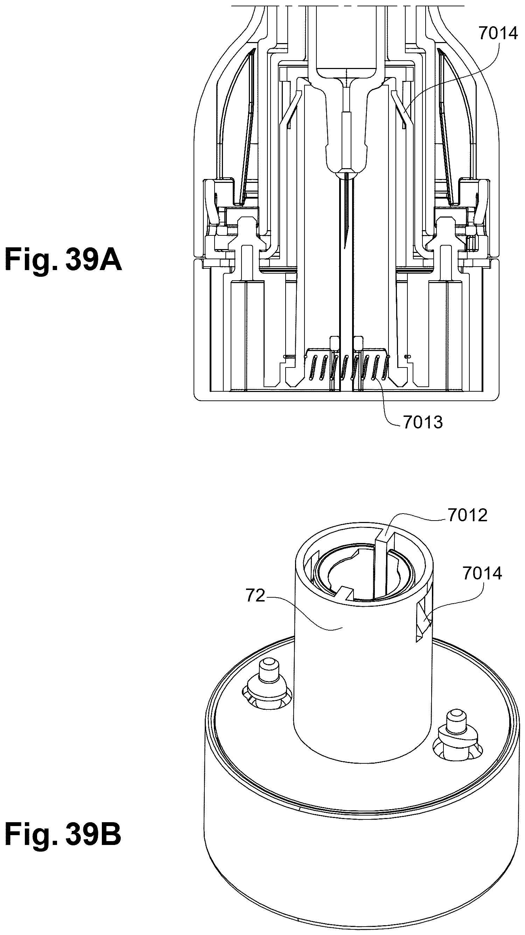

Figures 39A to 39B illustrate the injection device 1 according to another embodiment. In this embodiment, a tension spring 7013 may be arranged in the cap 7 for maintaining the reversible remover 8 in the removing position. Besides, the cap 7 may include two diametrically opposite resilient arms 7014 which may be arranged at a proximal end of the guiding member 72 for exerting a radial inward force on the removing members 88 of the reversible remover 8. The resilient arms 7014 are also configured for guiding and positioning the cap 7 with respect to the medical container 100. When the reversible remover 8 is moved to the release position, the resilient arms 7014 flex in an outward radial direction to allow release of the needle shield 104. The cap 7 includes the inner axial rib 7012 that stops the removing members 88 in the removing position.

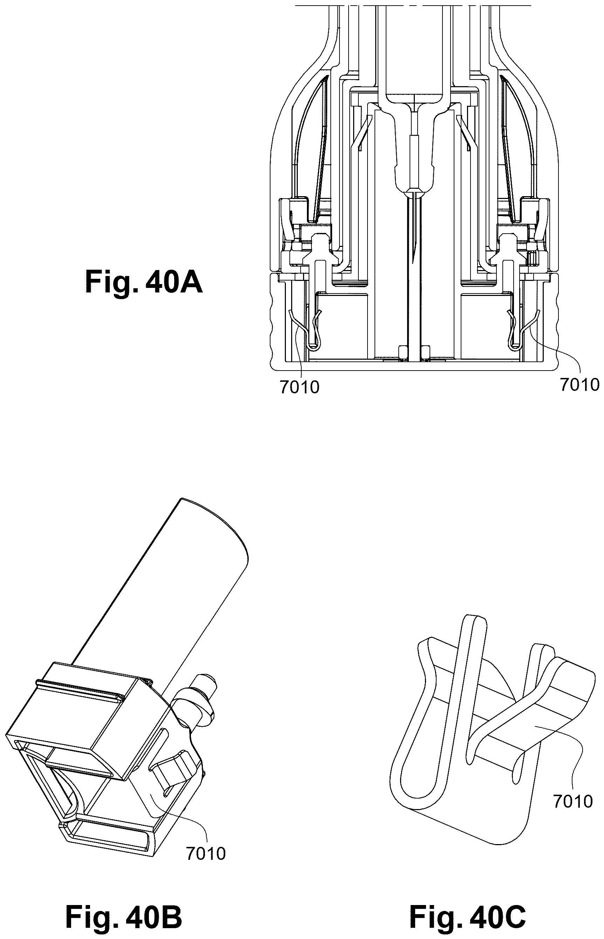

Figures 40A to 40C illustrate the injection device 1 according to another embodiment, which is similar to the embodiment of Figures 38A-38C, except that the spring blades 7010 here are distinct from the reversible remover 8 and are fixed to the reversible remover 8. The spring blades 7010 may be made of a metallic or plastic material.

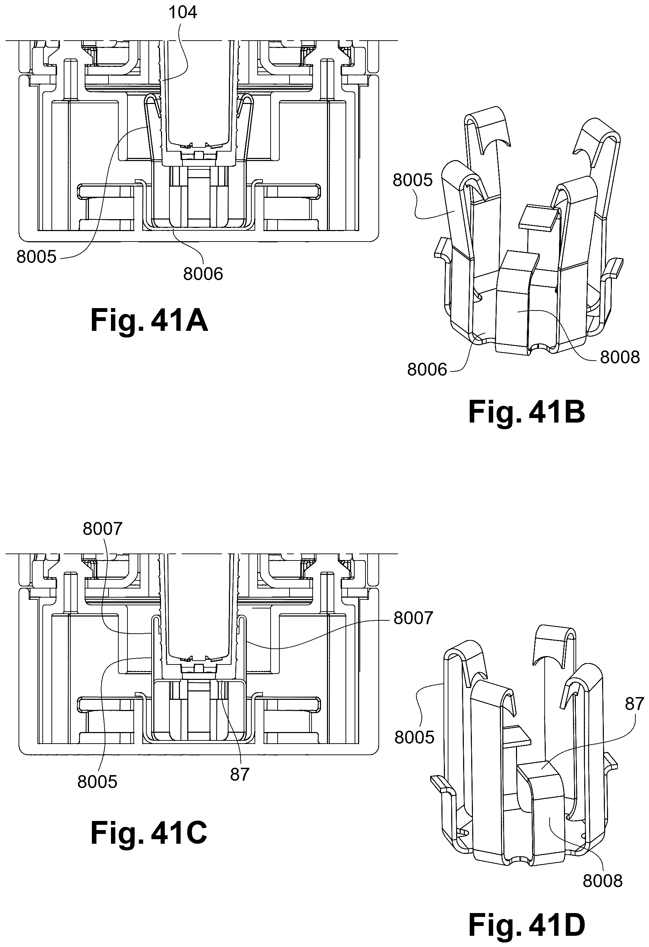

Figures 41A to 41D illustrate the injection device 1 according to another embodiment. The removing members 88 of the reversible removers 8 and the rest of the reversible removers 8 here are two separate components. The removing members 88 may be in the form of radial hooks arranged at a proximal end of resilient proximally protruding legs 8005 of a metallic claw 8006. The reversible remover 8 includes a pushing surface 8007 for radially inwardly pressing against the legs 8005 in order to move the removing members 88 to the releasing position, in which the radial hooks prick into the needle shield 104 such that subsequent removal of the cap 7 entails removal of the needle shield 104. Besides, the proximal stop 87 may be arranged at a proximal end of two diametrically opposite legs 8008 of the metallic claw 8006 for axially abutting against the distal end of the needle shield 104, thereby stopping insertion of the medical container 100 at a predetermined axial position.

It is contemplated that the spring blades 7010 of Figures 40A-40C or the legs 8005 of the metallic claw 8006 of Figures 41A-41D are configured for resiliently deforming, such that they move back to their initial shape after deformation. The spring blades 7010 or the legs 8005 of the metallic claw 8006 may be made for instance of nickel titanium alloy, such as nitinol.

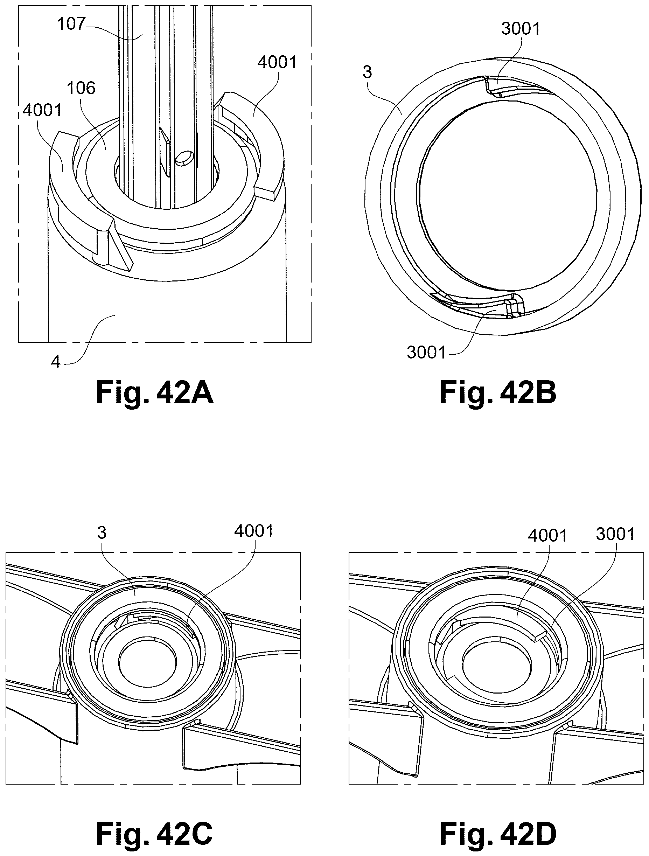

Figures 42A to 42D illustrate the injection device 1 according to another embodiment. In this embodiment, the medical container 100 may be a round flange syringe, i.e. may have a circular radial flange 106. Therefore, the holder 4 may have a retainer 4001 for axially retaining the medical container 100. The axial retainer 4001 may be in the form of circumferential tabs which may be arranged at a proximal end of the holder 4 and which are configured for radially deforming between a release position and a blocking position. Movement of the axial retainer 4001 from the release position to the blocking position is due to a cam 3001 which may be arranged at a proximal end of the locker 3 when the locker 3 rotates together with the cap 7. The retainer 4001 and the holder 4 may here be made of a single piece.

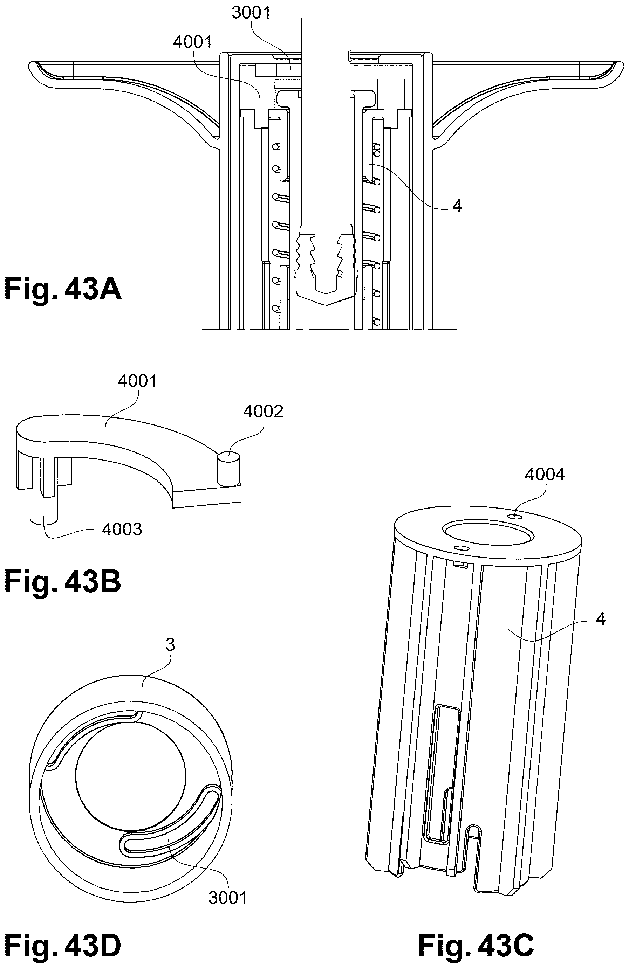

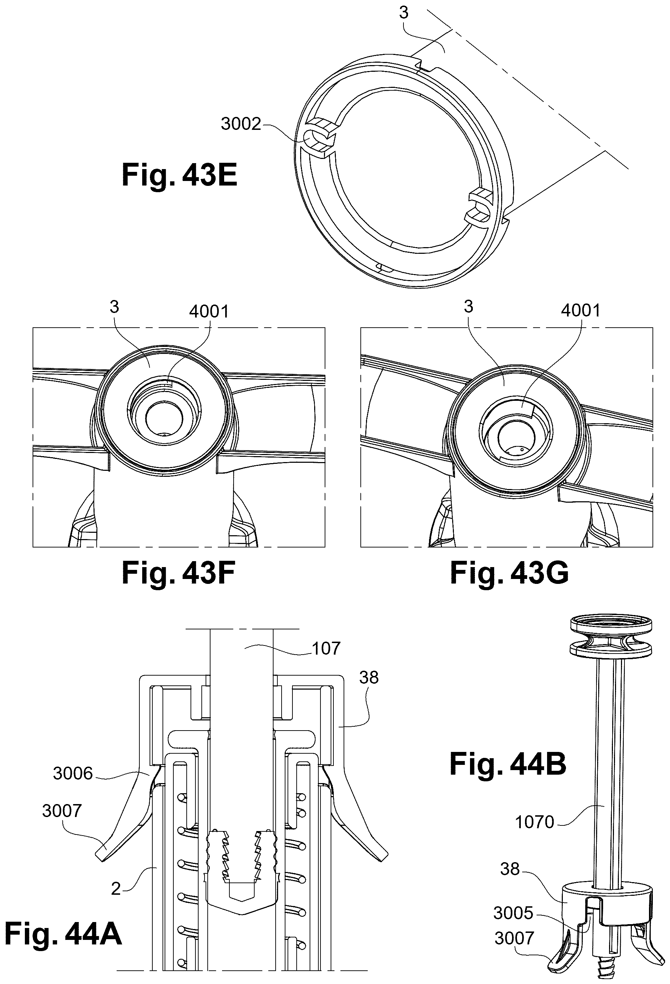

Figures 43A to 43G illustrate the injection device 1 according to another embodiment. The holder 4 includes the retainer 4001, but the retainer 4001 and the holder 4 are here made of two distinct parts. The retainer 4001 is rotatably mounted, via a mounting pin 4003 engaged in a bore 4004 of the holder 4, onto the proximal end of the holder 4, and includes a guiding pin 4002 for engaging a track that forms the cam 3002 of the locker 3. Rotation of the locker 3 accordingly causes radial inward movement of the retainer 4001 to lock the medical container 100 within the housing 10. The locker 3 may further include two clips or cavities 3002 for receiving the pin 81 of the reversible remover 8 such that rotation of the cap 7 is transmitted to the locker 3 via the engagement between the pin 81 and the corresponding cavity 3002 of the locker 3.

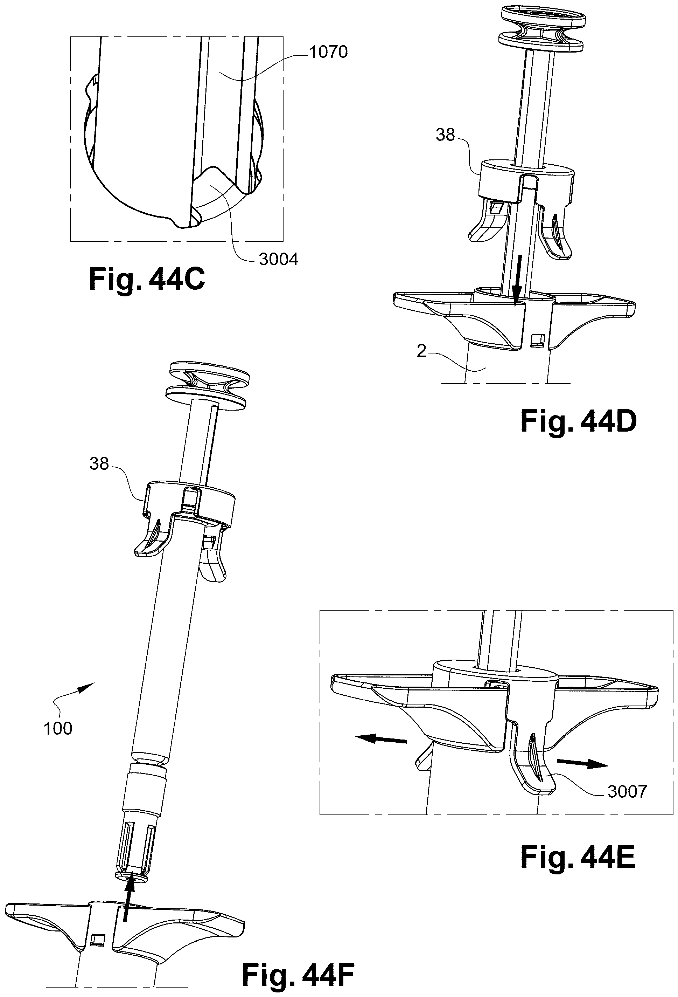

Figures 44A to 44F illustrate the injection device 1 according to another embodiment. Here, the locker 3 includes an upper ring 38 configured to be slidably mounted onto the plunger rod 107 and configured for being secured to the proximal end of the shell 2 in order to lock the holder 4 and the medical container 100 within the housing 10. The upper ring 38 includes an opening 3003 for receiving the plunger, a guiding feature 3004 such as a radial arm for engaging an axial slot 1070 of the plunger rod 107, notches 3005 for receiving the finger flange 21 of the shell 2, and may include a snap-fit element 3006 such as a distally extending resilient leg provided with an inner radial hook for engaging a snap-fit element 2001 of the shell 2, such as a lateral opening, so that the upper ring 38 is axially and rotationally secured to the shell 2. A removing element 3007, such as an oblique extension connected to the snap-fit element 3006, may help the user remove the upper ring 38 from the shell 2 in order to withdraw the used medical container 100. Due to the positioning of the upper ring 38 outside the shell 2, this embodiment allows for reducing the diameter of the shell 2, and thus the diameter of the injection device 1. The upper ring 38 here is rotationally fixed, but axially movable between the initial release position, Figure 44D, and the locking position, Figure 44E. The upper ring 38 is first assembled to the plunger rod 107, Figure 44B. After insertion of the barrel 101, the plunger rod 107 is inserted in the proximal end of the barrel 101, Figure 44D. The upper ring 38 is axially moved along the plunger rod 107 in the distal direction until the snap-fit elements 2001, 3006 engage such that the upper ring 38 is axially and rotationally fixed to the proximal end of the shell 2. After injection, the user can disengage the snap-fit elements 2001, 3006 of the upper ring 38 and the shell 2, Figure 44E, such that the upper ring 38 is removed together with the medical container 100, Figure 44F.

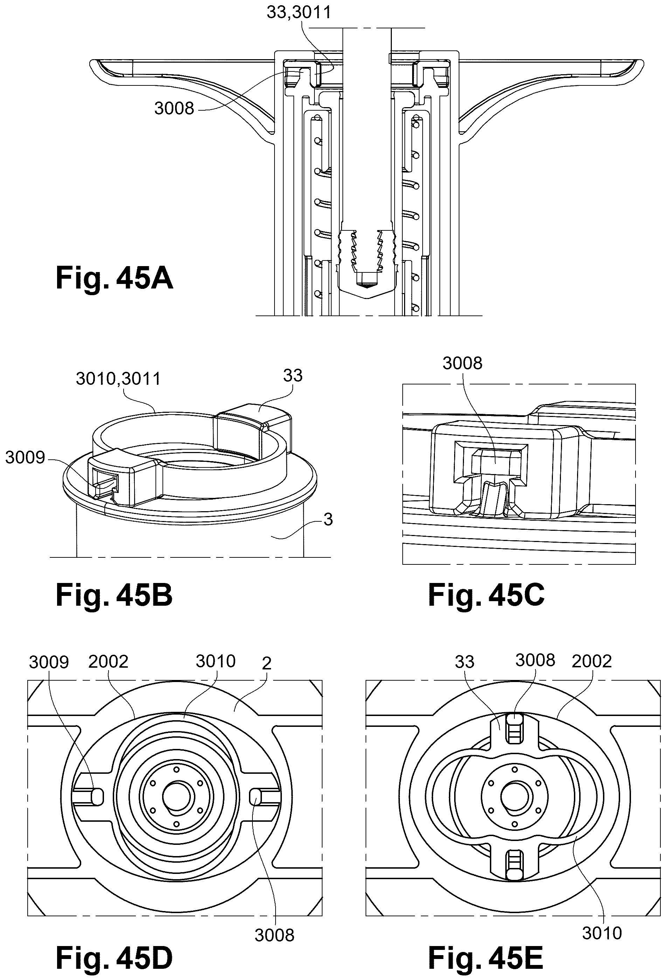

Figures 45A to 45E illustrate the injection device 1 according to another embodiment. In this embodiment, the shell 2 includes a cam 2002: for instance the proximal opening defined at the proximal end of the shell 2 may have a non-circular, such as an oval, ovoid or ellipsoidal shape. The cam 2002 of the shell 2 is configured for cooperating with the locking member 33 of the locker 3. The locking member 33 is radially movable between a release position, Figure 45D, allowing for insertion of the medical container 100 within the housing 10, and a locking position, Figure 45E, in which a distal shoulder 330 of the locking member 33 is configured for axially abutting against the medical container 100 and therefore prevents removal of the medical container 100. Movement of the locking member 33 from the release position to the locking position is caused by the locking member 33 radially abutting and sliding against the cam 2002 of the shell 2 when the locker 3 rotates together with the cap 7. The locker 3 may include a guiding pin 3008 proximally protruding from the proximal end of the locker 3 and engaging a radial slot 3009 of the locking member 33 such that the radial movement of the locking member 33 is guided by the guiding pin 3008. A resilient arm 3010 may be configured for moving the locking member 33 towards the release position when the cap 7 rotates together with the locker 3 in the opposite rotational direction. The locker 3 may include two diametrically opposite locking members 33 connected by two resilient arms 3010, and the locking members 33 and the resilient arms 3010 may be made of a single piece, thereby forming a locking ring 3011 arranged at the proximal end of the locker 3. The locking ring 3011 is assembled to the locker 3 via the guiding pins 3008 and the radial slots 3009, but the locking ring 3011 and the locker 3 may be two distinct parts.

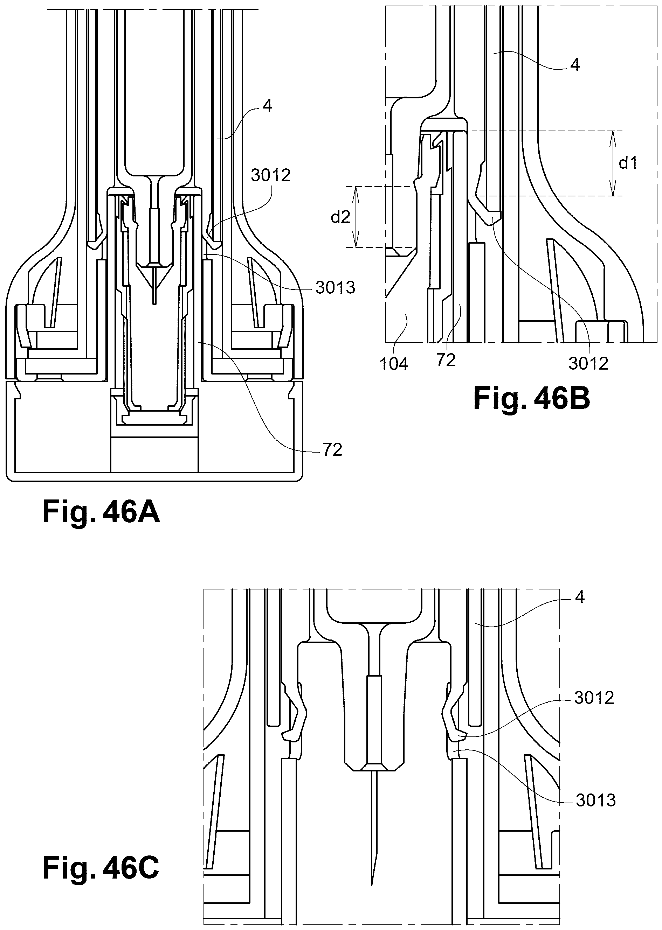

With reference to Figures 46A-46C, when the user removes the cap 7, the medical container 100 and the holder 4 indeed are slightly pulled in the distal direction due to the engagement between the needle shield 104 and the distal tip 102 of the medical container 100. As a result, the safety spring 5 is tensioned and when the cap 7 is eventually detached, the safety spring 5 suddenly pushes the holder 4 and the medical container 100 back in the proximal direction. This may cause the medical container 100 to hit the locker 3. The dampener 36 limits the impact. To avoid impact between the medical container 100 and the locker 3, the body 6 may include a resilient blocking element 3012, Figures 46A-46C, such as a resilient leg, which may distally extend in an opening 3013 through the sidewall of the body 6, for blocking distal movement of the holder 4, and thus of the medical container 100 when the cap 7 is being removed. The blocking element 3012 may act alternatively or complementarily to the dampener 36 and/or to the blocking element 34 of the locker 3. The blocking element 3012 may be radially movable between a blocking (deformed) position, Figure 46A, in which the blocking element 3012 extends on the axial path of the holder 4, and a release (rest) position, Figure 46C, in which the blocking element 3012 is away from the axial path of the holder 4 to allow injection. The blocking element 3012 may here be maintained in the blocking (deformed) position by the cap 7 radially abutting against the blocking element 3012. The blocking element 3012 may be located so as to be radially blocked by the cap 7 as long as the cap 7 exerts a distal pulling force on the medical container 100. Specifically, the axial distance d1 between the proximal end of the guiding member of the cap 7 and the blocking element 3012 may be equal to or greater than the axial distance d2 of friction between the needle shield 104 and the distal tip 102. Thus, once the cap 7 is detached, the blocking element 3012 automatically returns to the release position. Since the medical container 100 and the holder 4 have not distally moved during removal of the cap 7, there is no sudden movement of the medical container 100 back in the proximal direction.

It is readily understandable from the above description that the device of the invention makes it easier for the user to perform an injection operation and has the major advantage of being re-usable, thereby improving sustainability. It is to be understood that the present invention is not limited to the embodiments described above and illustrated in the drawings; rather, the skilled person will recognize that many changes and modifications may be made within the scope of the appended claims.

## Claims

1. Housing device (10) configured to receive a medical container (100) having an injection needle (103) and a needle shield (104) for sealing the injection needle (103), the housing device (10) including:
a body (6) extending along a longitudinal axis A, the body (6) delimiting an inner cavity for accommodating the medical container (100),
a cap (7) removably attached to the body (6), the cap (7) being rotationally movable around the longitudinal axis A with respect to the body (6) between an initial position, in which the cap (7) is axially locked to the body (6), and a release position, in which the cap (7) can distally move with respect to the body (6) in order to be removed,
a reversible remover (8) coupled to the cap (7), the reversible remover (8) including a removing member (88) configured for removing the needle shield (104) from the medical container (100) and releasing the needle shield (104) back onto the medical container (100), the removing member (88) being radially movable between a removing position, in which the removing member (88) is configured for removing the needle shield (104), and a release position, in which the removing member (88) releases the needle shield (104),
a cam element (61) arranged on the body (6) for cooperating with a cam member (85) of the remover (8) such that rotation of the cap (7) with respect to the body (6) from the initial position to the release position causes movement of the removing member (88) from the release position to the locking position, and rotation of the cap (7) with respect to the body (6) from the release position to the initial position causes movement of the removing member (88) from the locking position to the release position.

2. Housing device (10) according to the preceding claim, wherein the housing device (10) includes a holder (4) including a proximal abutment (42) configured for axially abutting against the medical container (100), the holder (4) being axially movable along the longitudinal axis A with respect to the body (6) between an initial position and an injection position, distally located with respect to the initial position.

3. Housing device (10) according to the preceding claim, wherein the holder (4) includes an injection depth controlling member (46) configured for axially abutting against the body (6) to stop distal movement of the holder (4) at a predetermined axial position.

4. Housing device (10) according to any one of the preceding claims 2 or 3, wherein the holder (4) includes an inner ring (402) for receiving the medical container 100, and the inner ring (402) has a distal end provided with a proximally tapered surface (47) for avoiding that the needle shield (104) catches on the inner ring (402) when the used medical container (100) is being removed from the housing device (10).

5. Housing device (10) according to any one of the preceding claims 2 to 4, wherein the housing device (10) includes a blocking element (34, 3012) movable between a blocking position in which the blocking element (34, 3012) axially abuts against the holder (4) to prevent distal movement of the holder (4) during the cap (7) removal, and a release position, in which the blocking element (34, 3012) allows for distal movement of the holder (4) with respect to the body (6).

6. Housing device (10) according to any one of the preceding claims, wherein the housing device (10) includes a locker (3) configured for axially locking the medical container (100) within the body (6).

7. Housing device (10) according to the preceding claim, wherein the locker (3) is movable with respect to the body (6) between an initial release position, in which the locker (3) allows for insertion of the medical container (100) within the body (6), and a locking position, in which the locker (3) prevents removal of the medical container (100) from the body (6).

8. Housing device (10) according to the preceding claim, wherein the locker (3) is rotationally movable between the initial release position and the locking position, and the housing device (10) includes a driving member (32) coupled to the cap (7) such that rotation of the cap (7) from the initial position to the release position causes rotation of the locker (3) from the release position to the locking position, and rotation of the cap (7) from the release position back to the locking position causes rotation of the locker (3) from the locking position back to the initial release position.

9. Housing device (10) according to the preceding claim, wherein the driving member (32) is arranged on the reversible remover (8).

10. Housing device (10) according to any one of the preceding claims 6 to 9, wherein the locker (3) includes a dampener (36) for reducing impact of the medical container (100) against the locker (3) when the cap (7) is removed from the body (6).

11. Housing device (10) according to any one of the preceding claims 6 to 10, wherein the housing device (10) includes one or more retaining members (31) configured to retain the locker (3) in the initial release position and in the locking position.

12. Housing device (10) according to any one of the preceding claims, wherein the housing device (10) includes a shell (2) configured for allowing a user to handle the housing device (10).

13. Housing device (10) according to any one of the preceding claims, wherein the housing device (10) includes a proximal stop (87) configured for axially abutting against the needle shield (104) in order to stop insertion of the medical container (100) at a predetermined axial position.

14. Housing device (10) according to any one of the preceding claims, wherein the housing device (10) includes snap-fit elements (770, 86) for maintaining the reversible remover (8) in the removing position.

15. Injection device (1) including a housing device (10) according to any one of the preceding claims and a medical container (100) arranged within the housing device (10).
